(19) Europäisches Patentamt
European Patent Office
Office européen des brevets

(11) Veröffentlichungsnummer: **0 007 643**
**B1**

(12) # EUROPÄISCHE PATENTSCHRIFT

(45) Veröffentlichungstag der Patentschrift: **15.02.84**

(21) Anmeldenummer: **79102727.9**

(22) Anmeldetag: **31.07.79**

(51) Int. Cl.³: **C 07 D 498/04,** A 61 K 31/53, C 07 D 251/66, C 07 D 401/04 // C07D211/98 ,(C07D498/04, 271/00, 251/00)

(54) **Oxadiazolotriazinderivate.**

(30) Priorität: **31.07.78 CH 8184/78**
**15.05.79 CH 4505/79**

(43) Veröffentlichungstag der Anmeldung: **06.02.80 Patentblatt 80/3**

(45) Bekanntmachung des Hinweises auf die Patenterteilung: **15.02.84 Patentblatt 84/7**

(84) Benannte Vertragsstaaten: **AT BE CH DE FR GB IT LU NL SE**

(56) Entgegenhaltungen:
**DE - A - 1 620 637**
**DE - A - 2 804 519**
**US - A - 3 270 014**

**Schröder et al., Arzneimittelchemie I, (Thieme-Verlag Stuttgart 1976) S. 33**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Muller, Jean-Claude Dr., 27 rue Albert Schweitzer, Parc d'Entremont, F-68170 Rixheim (FR)**
Erfinder: **Ramuz, Henri Dr., Rheinparkstrasse 3, CH-4127 Birsfelden (CH)**

(74) Vertreter: **Lederer, Franz, Dr. et al, Patentanwälte Lederer, Meyer Lucile-Grahn-Strasse 22, D-8000 München 80 (DE)**

EP 0 007 643 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Oxadiazolotriazinderivate

Die vorliegende Erfindung betrifft neue Oxadiazolotriazinderivate der allgemeinen Formel

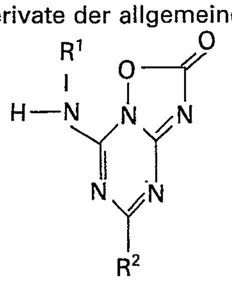

I

worin $R^1$ Wasserstoff oder eine der Gruppen $-\overset{O}{\overset{\|}{C}}-R^3$, $-COOR^4$ und $-CH(R^5)COOR^6$ bedeutet und $R^2$ Diallylamino oder 1,2,5,6-Tetrahydropyridin-1-yl, $R^3$ Alkyl, Haloalkyl, Alkoxyalkyl, Aryloxyalkyl, Arylalkyloxyalkyl, Arylalkyl, Alkoxycarbonylalkyl, Aryl, die Gruppe $-C(R^7)=C(R^8)(R^9)$, Alkoxycarbonylalkylcarbonyl oder gegebenenfalls über eine Methylengruppe gebundenes Pyridyl, Furyl oder Thienyl, $R^4$ Alkyl, Alkoxyalkyl, Arylalkyl, Aryl oder Allyl, $R^5$ Wasserstoff oder Alkyl, $R^6$ Alkyl, $R^7$ und $R^8$ je Wasserstoff, Alkyl, Aryl, Arylalkyl, Pyridyl, Furyl oder Thienyl und $R^9$ Wasserstoff oder Methyl bedeuten, wobei die Alkyl- und Alkoxyreste 1–8 Kohlenstoffatome aufweisen und der Arylrest – allein oder in Kombination – einen ein- oder zweikernigen aromatischen Rest mit bis zu 12 Kohlenstoffatomen bedeutet, in welchem eines oder mehrere der Wasserstoffatome durch Alkyl, Alkoxy, Halogen, Cyano, Nitro, Alkoxycarbonyl oder $C_1$–$C_8$-Alkanoyloxy ersetzt sein können, sowie Salze davon.

Aus der US Patentschrift No. 3270014 sind 1,2-Dihydro-1-hydroxy-1,3,5-triazinderivate, u.a. das 1,2-Dihydro-1-hydroxy-6-amino-4-diallylamino-2-imino-1,3,5-triazin bekannt, welche am Sauerstoffatom der 1-Hydroxygruppe und/oder am Stickstoffatom der 2-Iminogruppe durch Carboxyacyl substituiert sein können. Die carboxyacylierten Derivate besitzen blutdrucksenkende und vasodilatierende Eigenschaften. In der Deutschen Offenlegungsschrift Nr. 2804519, welche am 10. August 1978 offengelegt wurde, sind Oxadiazolopyrimidinderivate, u.a. das Äthyl-5-dimethylamino-2-oxo-2H-[1,2,4]oxadiazolo[2,3-a]pyrimidin-7-carbamat, und deren blutdrucksenkende und vasodilatierenden Eigenschaften beschrieben.

Der in dieser Beschreibung verwendete Ausdruck «Alkyl» – allein oder in Kombination – bezieht sich auf geradkettige und verzweigte, gesättigte Kohlenwasserstoffreste mit 1–8 Kohlenstoffatomen, wie z.B. Methyl, Äthyl, n-Propyl, Isopropyl, n-Butyl, Isobutyl, tert. Butyl und dgl. «Alkoxy» bezieht sich auf Alkyläthergruppen, in denen der Ausdruck «Alkyl» die obige Bedeutung besitzt. Der Ausdruck «Haloalkyl» bezieht sich auf Alkylgruppen, in denen eines oder mehrere der Wasserstoffatome durch «Halogen» ersetzt ist. Der Ausdruck «Halogen» umfasst die vier Halogene Fluor, Chlor, Brom oder Jod. Der Ausdruck «Aryl» bezieht sich auf ein- oder zweikernige aromatische Reste mit bis zu 12 Kohlenstoffatomen, in denen eines oder mehrere der Wasserstoffatome durch Alkyl, Alkoxy, Halogen, Cyano, Nitro, Alkoxycarbonyl oder $C_1$–$C_8$-Alkanoyloxy ersetzt sein können, wie z.B. Phenyl, Halophenyl, Dihalophenyl, Methoxyphenyl, Dimethoxyphenyl, Nitrophenyl, Tolyl, Methoxycarbonylphenyl, Naphthyl und dgl. Der Ausdruck «Aryloxy» bezieht sich auf Aryläthergruppen, in denen der Ausdruck «Aryl» die obige Bedeutung besitzt. Der Ausdruck «Arylalkyl» betrifft Arylalkylgruppen, wie Benzyl, Phenäthyl und dgl. «Arylalkyloxy» betrifft Arylalkyläthergruppen, wie Benzyloxy, Phenäthyloxy und dgl. Der Ausdruck «$C_1$–$C_8$-Alkanoyloxy» betrifft den Acyloxyteil einer Alkancarbonsäure. Der Alkylteil der Alkancarbonsäure enthält 1–7, besonders bevorzugt 1–3 Kohlenstoffatome. Der Acylteil der Ameisensäure soll jedoch auch erfasst werden. Es handelt sich also um Reste, wie Formyloxy, Acetyloxy, Propionyloxy, Butyryloxy und dgl.

Von den Verbindungen der Formel I sind diejenigen bevorzugt, in denen $R^2$ Diallylamino bedeutet. Weiters sind diejenigen Verbindungen der Formel I bevorzugt, in denen $R^1$ Wasserstoff oder die Gruppe $-\overset{O}{\overset{\|}{C}}-R^3$ bedeuten. Besonders bevorzugt sind diejenigen, in denen $R^3$ Aryl, Alkoxyalkyl, die Gruppe $-C(R^7)=C(R^8)(R^9)$ oder gegebenenfalls über eine Methylengruppe gebundenes Pyridyl, Furyl oder Thienyl bedeutet. Ganz besonders bevorzugt sind diejenigen, in denen $R^3$ Phenyl, Methoxymethyl, Äthoxymethyl, die Gruppe $-C(CH_3)=CH_2$ oder $-CH=C(CH_3)_2$ oder Furyl bedeutet.

Aus dem obigen folgt, dass diejenigen Verbindungen der Formel I besonders bevorzugt sind, in denen $R^2$ Diallylamino, $R^1$ Wasserstoff oder die Gruppe $-\overset{O}{\overset{\|}{C}}-R^3$ und $R^3$ Phenyl, Methoxymethyl, Äthoxymethyl, die Gruppe $-C(CH_3)=CH_2$ oder $-CH=C(CH_3)_2$ oder Furyl bedeuten.

Ganz speziell bevorzugt sind:

N-(5-Diallylamino-2-oxo-2H-[1,2,4]oxadiazolo[2,3,-a]-s-triazin-7-yl)benzamid,

N-(5-Diallylamino-2-oxo-2H-[1,2,4]oxadiazolo[2,3-a]-s-triazin-7-yl)-2-methoxyacetamid,

2-Äthoxy-N-(5-diallylamino-2-oxo-2H-[1,2,4]oxadiazolo[2,3-a]-s-triazin-7-yl)acetamid,

N-(5-Diallylamino-2-oxo-2H-[1,2,4]oxadiazolo[2,3-a]-s-triazin-7-yl)-2-methylacrylamid,

N-(5-Diallylamino-2-oxo-2H-[1,2,4]oxadiazolo[2,3-a]-s-triazin-7-yl)-2-furamid,

7-Amino-5-diallylamino-2H-[1,2,4]oxadiazolo[2,3-a]-s-triazin-2-on,

N-(5-Diallylamino-2-oxo-2H-[1,2,4]oxadiazolo[2,3-a]-s-triazin-7-yl)-3-methylcrotonamid.

Die Verbindungen der Formel I sowie deren

Salze können erfindungsgemäss so hergestellt werden, dass man

a) eine Verbindung der allgemeinen Formel

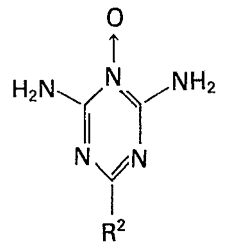

II

worin $R^2$ die in Anspruch 1 genannte Bedeutung besitzt, mit Phosgen umsetzt und gegebenenfalls eine so erhaltene Verbindung der allgemeinen Formel

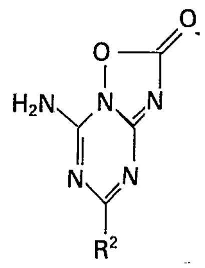

Ia

worin $R^2$ die obige Bedeutung besitzt, mit einem die Gruppe 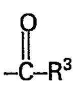 abgebenden Acylierungsmittel, wobei $R^3$ die in Anspruch 1 genannte Bedeutung besitzt, mit einem Chlorameisensäureester der allgemeinen Formel

$Cl-COOR^4$ III

worin $R^4$ die in Anspruch 1 genannte Bedeutung besitzt, oder mit einer Verbindung der allgemeinen Formel

$XCH(R^5)COOR^6$ IV

worin X Halogen bedeutet und $R^5$ und $R^6$ die in Anspruch 1 genannte Bedeutung besitzen, umsetzt, oder

b) zur Herstellung von Verbindungen der Formel I, worin $R^1$ die Gruppe $-COOR^4$ bedeutet und $R^2$ die obige Bedeutung besitzt, eine Verbindung der allgemeinen Formel

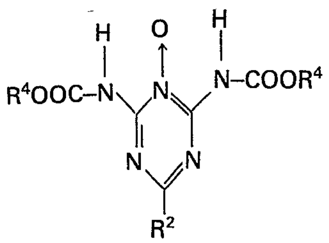

V

worin $R^2$ und $R^4$ die obige Bedeutung besitzen, cyclisiert, und

c) erwünschtenfalls eine erhaltene Verbindung der Formel I in ein Salz oder ein Salz in ein anderes Salz überführt.

Die Umsetzung einer Verbindung der Formel II mit Phosgen erfolgt in an sich bekannter Weise in Gegenwart eines Lösungsmittels oder Lösungsmittelgemisches. Als Lösungsmittel kommen aromatische Kohlenwasserstoffe, wie Benzol, Toluol oder Xylol, chlorierte Kohlenwasserstoffe, wie Methylenchlorid oder Chloroform, Dimethylformamid, und dgl. oder Gemische davon in Frage. Die Reaktion wird zweckmässig bei Atmosphärendruck und einer Temperatur zwischen etwa −20° und 50°C, vorzugsweise zwischen etwa 0°C und der Raumtemperatur, in Gegenwart eines säurebindenden Mittels durchgeführt. Geeignete säurebindende Mittel sind tertiäre organische Basen, wie Triäthylamin, Äthyldiisopropylamin oder Pyridin, oder anorganische Basen, wie Alkalihydroxyde, z.B. Natriumhydroxyd oder Kaliumhydroxyd, Erdalkalihydroxyde, z.B. Bariumhydroxyd oder Calciumhydroxyd, Carbonate, z.B. Kaliumcarbonat oder Natriumbicarbonat, Bicarbonate, z.B. Natriumcarbonat, und dgl.

Die gegebenenfalls anschliessende Acylierung einer erhaltenen Verbindung der Formel Ia erfolgt ebenfalls nach an sich bekannten Methoden. Geeignete, die Gruppe 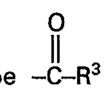 abgebende Acylierungsmittel sind insbesondere aktivierte Säurederivate, wie Säurehalogenide oder Säureanhydride der Formeln $R^3-COX$ und $(R^3-CO)_2O$. Die Reaktion wird in einem inerten Lösungsmittel oder Lösungsmittelgemisch bei einer Temperatur zwischen etwa 0° und 70°C, vorzugsweise zwischen etwa 0° und 30°C, besonders bevorzugt bei Raumtemperatur, in Gegenwart eines säurebindenden Mittels durchgeführt. Als Lösungsmittel kommen insbesondere aromatische Kohlenwasserstoffe, wie Benzol, Toluol oder Xylol, chlorierte Kohlenwasserstoffe, wie Methylenchlorid oder Chloroform, Äther, wie Tetrahydrofuran oder Dioxan, Dimethylformamid, Dimethylsulfoxid, Hexamethylphosphorsäuretriamid und dgl. in Frage. Geeignete säurebindende Mittel sind tertiäre organische Basen, wie Triäthylamin, Äthyldiisopropylamin oder Pyridin, oder anorganische Basen wie Alkalihydroxyde, z.B. Natriumhydroxyd oder Kaliumhydroxyd, Erdalkalihydroxyde, z.B. Bariumhydroxyd oder Calciumhydroxyd, Carbonate, z.B. Kaliumcarbonat oder Natriumcarbonat, Bicarbonate, z.B. Natriumbicarbonat, und dgl.

Die Umsetzung mit einem Chlorameisensäureester der Formel III erfolgt in einem inerten Lösungsmittel oder Lösungsmittelgemisch in Gegenwart eines säurebindenden Mittels. Für den vorliegenden Zweck geeignete Lösungsmittel sind chlorierte Kohlenwasserstoffe, wie Methylenchlorid oder Chloroform, Äther, wie Diäthyläther, Tetrahydrofuran oder Dioxan, Dimethylformamid und dgl. oder Gemische davon. Die Umsetzung kann auch in einem wasserhaltigen Lösungsmittel bzw. in Gegenwart von Wasser in einem Zweiphasensystem wie z.B. Methylenchlorid/Wasser durchgeführt werden. Als säurebindende Mittel kommen Basen, wie Triäthylamin, Äthyldiisopropylamin, Dimethylamin, Pyridin, Alkalihydroxyde und dgl. in Frage. Wird die Umsetzung in Gegenwart einer flüssigen Base durchgeführt, so kann diese auch als Lösungsmittel dienen. Die Umsetzung wird zweckmässig bei Temperaturen zwischen etwa −10°C und Raumtemperatur durchgeführt, vorzugsweise zwischen etwa 0° und 10°C.

Die Umsetzung einer Verbindung der Formel Ia mit einer Verbindung der Formel IV erfolgt nach an sich bekannten Methoden in einem inerten Lösungsmittel, wie Aceton, Methanol, Tetrahydrofuran, Dimethylformamid, Hexamethylphosphorsäuretriamid und dgl. in Gegenwart eines säurebindenden Mittels, wie Kaliumcarbonat, Natriumcarbonat, Triäthylamin, Äthyldiisopropylamin und dgl. bei einer Temperatur zwischen etwa 0° und 50°C, vorzugsweise bei der Raumtemperatur. In einer bevorzugten Ausführungsform wird die Umsetzung in Aceton in Gegenwart von Kaliumcarbonat bei Raumtemperatur durchgeführt.

Die Cyclisation einer Verbindung der Formel V erfolgt in an sich bekannter Weise durch Erhitzen auf eine Temperatur zwischen etwa 50° und 200°C, vorzugsweise zwischen etwa 100° und 150°C. Die Reaktion kann in Abwesenheit oder Gegenwart eines Lösungsmittels oder Lösungsmittelgemisches durchgeführt werden. Wird die Reaktion in einem Lösungsmittel bzw. Lösungsmittelgemisch durchgeführt, so kommen als Lösungsmittel aromatische Kohlenwasserstoffe, wie Benzol, Toluol oder Xylol, chlorierte Kohlenwasserstoffe, wie Chloroform, Alkohole, wie Butanol oder Isobutanol, Äther, wie Dibutyläther, Dioxan oder Diäthylenglykoldimethyläther, Dimethylformamid, Dimethylsulfoxid und dgl. oder Gemische davon in Frage. Es ist klar, dass man entweder ein Lösungsmittel verwenden kann, dessen Siedepunkt höher liegt als die Reaktionstemperatur, oder dass man ein im weiter oben angegebenen Temperaturbereich siedendes Lösungsmittel bei dessen Rückflusstemperatur verwenden kann. Vorzugsweise wird die Reaktion unter Verwendung von Dimethylformamid oder Toluol als Lösungsmittel durchgeführt. Die Reaktionszeit hängt von der verwendeten Reaktionstemperatur ab und liegt zwischen etwa ¼ und 18 Stunden. Arbeitet man im bevorzugten Temperaturbereich zwischen etwa 100° und 150°C, so beträgt die Reaktionszeit etwa ¼ bis 12 Stunden, vorzugsweise ¼ bis 2 Stunden. Verwendet man einen Alkohol als Lösungsmittel, so ist es klar, dass man – will man keine Umesterung herbeiführen – den Alkohol verwenden muss, der der Alkohol-Komponente im verwendeten Ausgangsmaterial entspricht. In einer anderen, besonders bevorzugten Ausführungsform wird die Reaktion in Gegenwart einer Base durchgeführt, wodurch die Reaktionstemperatur wesentlich tiefer gehalten werden kann. In diesem Falle arbeitet man bevorzugt bei einer Temperatur zwischen etwa 0° und 80°C, zweckmässig bei der Raumtemperatur. Geeignete Basen sind anorganische Basen, wie Alkalihydroxyde, z.B. Natriumhydroxyd oder Kaliumhydroxyd, Erdalkalihydroxyde, z.B. Bariumhydroxyd oder Calciumhydroxyd, Carbonate, z.B. Kaliumcarbonat oder Natriumcarbonat, oder Bicarbonate, z.B. Natriumbicarbonat, oder organische Basen, wie Dimethylamin, Triäthylamin, Äthyldiisopropylamin und dgl. Arbeitet man in Gegenwart einer Base, so wird die Reaktion in einem geeigneten inerten Lösungsmittel oder Lösungsmittelgemisch durchgeführt. Als Lösungsmittel kommen die weiter oben genannten Lösungsmittel in Frage. Verwendet man eine anorganische Base, so arbeitet man zweckmässig in einem wasserhaltigen Lösungsmittelgemisch bzw. in Gegenwart von Wasser in einem Zweiphasensystem, wie z.B. Methylenchlorid/Wasser. Will man die weiter oben erwähnte Umesterung mit Absicht herbeiführen, so arbeitet man vorzugsweise in Gegenwart einer Base.

Die Ausgangsstoffe der Formel II sind teilweise bekannt und teilweise neu. So ist diejenige Verbindung, in der $R^2$ Diallylamino bedeutet, bekannt. Die Verbindung der Formel II, in der $R^2$ 1,2,5,6-Tetrahydropyridin-1-yl bedeutet, ist neu. Sie kann in Analogie zur Herstellung der bekannten Verbindung hergestellt werden. Zwei Herstellungsverfahren sind im nachfolgenden Schema I skizziert. Bezüglich der genauen Reaktionsbedingungen wird auf den Beispielteil verwiesen.

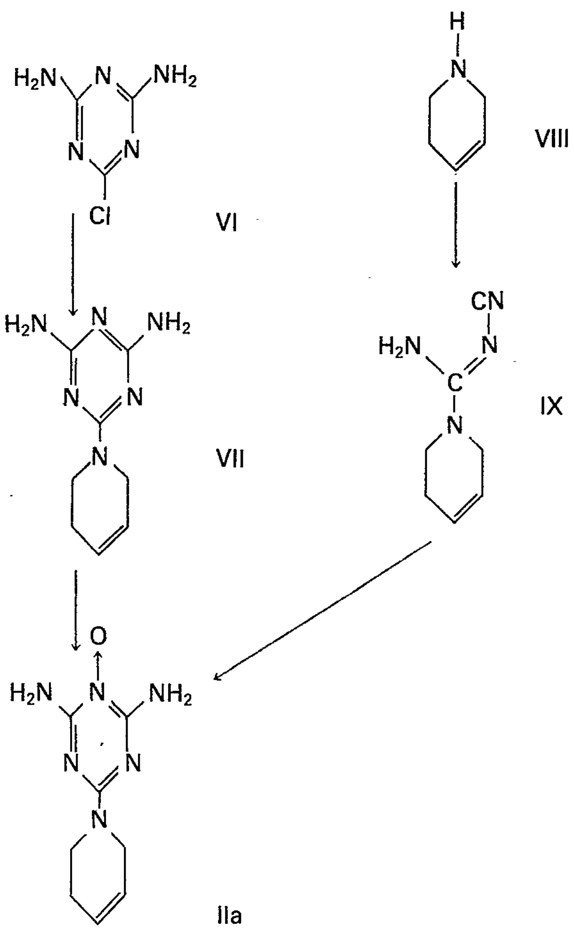

VI VIII VII IX IIa

Die Verbindungen der Formel V sind neu und können durch Umsetzen einer Verbindung der Formel II mit einem Chlorameisensäureester der Formel III hergestellt werden. Die Umsetzung erfolgt unter den für die Umsetzung der Verbindungen der Formel Ia mit einem Chlorameisensäureester der Formel III angegebenen Reaktionsbedingungen.

Die Verbindungen der Formel I können in Salze übergeführt werden, beispielsweise durch Behandlung mit einer anorganischen Base, wie einem Alkalihydroxyd, beispielsweise Natriumhydroxyd oder Kaliumhydroxyd, einem Erdalkalihydroxyd, beispielsweise Calciumhydroxyd, oder mit einer organischen Base, wie einem Monoalkylamin, z.B. Methylamin, einem Dialkylamin, z.B. Dimethylamin, einem Trialkylamin, z.B. Triäthylamin, einer basischen Aminosäure, z.B. Arginin, Piperidin, einem Azabicyclooctan- oder -nonan, z.B. 3-Azabicyclo[3.2.1.]octan oder 3-Azabicyclo[3.2.2.]nonan und dgl. Salze der Verbindungen der Formel I können auch durch Umsalzen eines geeigneten Salzes hergestellt werden. Von den Salzen der Verbindungen der Formel I sind die pharmazeutisch verwendbaren bevorzugt.

Die Verbindungen der Formel I sowie deren Salze besitzen langanhaltende wertvolle gefässerweiternde und/oder blutdrucksenkende Eigenschaften und können somit zur Behandlung gefässbedingter Hypertonien oder auch als Vasodilatatoren bei peripheren Durchblutungsstörungen Anwendung finden.

Die blutdrucksenkende Wirkung kann nach folgender Methode an wachen, weiblichen Hunden bestimmt werden:

Der arterielle Blutdruck wird oszillometrisch und die Herzfrequenz palpatorisch an einer chirurgisch vorbereiteten Carotisschlinge gemessen. Nach Bestimmung der Basiswerte wird die zu prüfende Substanz mit einer Magensonde den Hunden verabreicht, welche die Nacht über gefastet haben. Die einzelnen Dosen werden kummulativ im Abstand von je einem Tag verabreicht. Der arterielle Blutdruck und die Herzfrequenz werden 0,5, 1, 1,5, 3, 6 und 22 Stunden nach jeder Applikation gemessen. Die Versuchstiere werden während der Versuchsdauer sowie noch 2 Tage über den Zeitpunkt der letzten Substanzgabe hinaus auf ihre Verhaltensweise beobachtet.

Die blutdrucksenkende Wirkung kann auch nach folgender Methode an wachen, spontan hypertonen Ratten bestimmt werden:

Der systolische Blutdruck und die Herzfrequenz werden vor Substanzgabe zweimal gemessen. Die Substanzgabe erfolgt mittels Schlundsonde zweimal täglich, morgens und nachmittags. Beide Parameter werden 1, 3, 6 und 24 Stunden nach der Applikation gemessen und die prozentualen Veränderungen zu den Kontrollwerten berechnet. Der systolische Blutdruck wird indirekt an der Schwanzarterie der Ratte nach der Methode von Gerold et al. gemessen (Helv. Physiol. Acta 24: 58–69, 1966; Arzneimittelforschung 18: 1285–1287, 1968).

In den nachfolgenden Tabellen sind die erhaltenen Resultate von N-(5-Diallylamino-2-oxo-2H-[1,2,4]-oxadiazolo-[2,3-a]-s-triazin-7-yl)benzamid zusammengestellt, wobei jeweils die Mittelwerte aus 5 Versuchen angegeben sind.

Tabelle I

| Dosis | Stunden nach Substanzgabe | | | | | | |
|---|---|---|---|---|---|---|---|
| mg/kg | 0[1] | 0.5 | 1.0 | 1.5 | 3.0 | 6.0 | 22 |
| p.o. | Blutdruck △% (mm Hg) | | | | | | |
| 1.0 | 135 ± 1.3 | − 2.2 ± 1.5 | − 3.2 ± 2.8 | − 2.2 ± 1.5 | − 3.2 ± 3.8 | − 3.2 ± 2.6 | − 3.2 ± 2.1 |
| 3.0 | 135 ± 1.3 | − 8.2 ± 5.7 | − 9.2 ± 6.4 | − 8.2 ± 6.3 | − 8.2 ± 5.5 | − 10.2 ± 3.5 | − 8.0 ± 3.0 |
| 10.0 | 135 ± 1.3 | − 15.2 ± 3.4 | − 18.2 ± 3.8 | − 20.2 ± 5.0 | − 22.2 ± 6.4 | − 15.2 ± 5.5 | − 13.2 ± 4.0 |
| 30.0 | 135 ± 1.3 | − 21.2 ± 1.3 | − 22.2 ± 2.3 | − 26.2 ± 1.9 | − 21.2 ± 3.7 | − 18.2 ± 4.4 | − 13.8 ± 1.5 |
| | Herzfrequenz △% | | | | | | |
| 1.0 | 80 ± 1.8 | 2.2 ± 3.2 | 3.0 ± 2.3 | 1.8 ± 3.0 | 3.8 ± 2.2 | 7.0 ± 6.3 | 14.8 ± 9.9 |
| 3.0 | 80 ± 1.8 | 21.8 ± 10.1 | 27.0 ± 14.6 | 26.2 ± 14.3 | 29.0 ± 13.6 | 43.4 ± 16.3 | 51.0 ± 7.9 |
| 10.0 | 80 ± 1.8 | 56.2 ± 6.1 | 56.5 ± 5.6 | 62.5 ± 5.0 | 65.8 ± 6.3 | 60.5 ± 14.7 | 69.2 ± 2.3 |
| 30.0 | 80 ± 1.8 | 72.2 ± 7.7 | 71.0 ± 9.6 | 71.8 ± 8.2 | 72.2 ± 6.9 | 73.4 ± 7.5 | 57.0 ± 9.8 |

[1] Basiswert

Tabelle II

| Dosis mg/kg p.o. | △% Blutdruck | △% Herzfrequenz | Wirkungsdauer in Stunden |
|---|---|---|---|
| 1.0 | − 15.0% | + 7.2% | > 24 Std. |
| 3.0 | − 21.6% | + 7.1% | > 24 Std. |
| 10.0 | − 32.8% | + 22.1% | > 24 Std. |
| 30.0 | − 45.0% | + 26.5% | > 24 Std. |

Die Verfahrensprodukte und deren pharmazeutisch verwendbaren Salze können als Heilmittel

z. B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z. B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z. B. als Tabletten, Dragées, Suppositorien, Kapseln oder in flüssiger Form, z. B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes oder Puffer. Sie können auch noch andere therapeutisch wertvolle Stoffe enthalten.

Die tägliche Dosis bei oraler Verabreichung liegt zwischen etwa 10 und 500 mg; bei i.v.-Verabreichung zwischen etwa 1 und 50 mg. Die angegebenen Dosierungen sind jedoch nur als Beispiele zu verstehen und können je nach der Schwere des zu behandelnden Falles und nach Ermessen des behandelnden Arztes abgeändert werden.

Die nachfolgenden Beispiele erläutern die Erfindung. Alle Temperaturen sind in Celsiusgraden angegeben. Die Schmelzpunkte sind nicht korrigiert.

Beispiel 1

75 g (0,337 Mol) 2,4-Diamino-6-diallylamino-s-triazin-3-oxid werden in 800 ml Methylenchlorid suspendiert und unter Rühren auf 0° abgekühlt. Bei dieser Temperatur werden dann gleichzeitig 180 ml (0,346 Mol) 20%iges Phosgen in Toluol und soviel 10%ige Natronlauge innerhalb von 45 Minuten zugetropft, dass der pH bei 7–7,5 gehalten wird. Das Gemisch wird anschliessend noch während 1 Stunde gerührt. Danach gibt man Methylenchlorid und Methanol zu, um das ausgefallene Produkt zu lösen. Die beiden Phasen werden getrennt, und die wässrige Phase wird noch zweimal mit Methylenchlorid extrahiert. Die kombinierten organischen Extrakte werden über Magnesiumsulfat getrocknet und unter vermindertem Druck eingedampft, wobei man 7-Amino-5-diallylamino-2H-[1,2,4]oxadiazolo[2,3-a]-s-triazin-2-on vom Schmelzpunkt 211–213° erhält.

Beispiel 2

6 g (0,016 Mol) Diäthyl-6-diallylamino-s-triazin-2,4-dicarbamat-3-oxid werden in 120 ml Dimethylformamid suspendiert und während 20 Minuten unter Argonatmosphäre und Rühren auf 140° erwärmt. Das Lösungsmittel wird danach unter vermindertem Druck abgedampft, und der Rückstand aus Methylenchlorid/Äthanol/Diäthyläther umkristallisiert, wobei man reines Äthyl 5-diallylamino-2-oxo-2H-[1,2,4]oxadiazolo[2,3-a]-s-triazin-7-carbamat vom Schmelzpunkt 172–174° erhält.

In analoger Weise erhält man

aus 4,4 g (0,013 Mol) Dimethyl 6-diallylamino-s-triazin-2,4-dicarbamat-3-oxid das Methyl 5-diallylamino-2-oxo-2H-[1,2,4]oxadiazolo[2,3-a]-s-triazin-7-carbamat, Smp. 184–185°;

aus 5,9 g (0,014 Mol) Diisobutyl 6-diallylamino-s-triazin-2,4-dicarbamat-3-oxid das Isobutyl 5-diallylamino-2-oxo-2H-[1,2,4]oxadiazolo[2,3-a]-s-triazin-7-carbamat, Smp. 160–162°.

Das als Ausgangsmaterial verwendete Diäthyl 6-diallylamino-s-triazin-2,4-dicarbamat-3-oxid kann wie folgt hergestellt werden:

5,0 g (0,0225 Mol) 2,4-Diamino-6-diallylamino-s-triazin-3-oxid werden in 200 ml Methylenchlorid und 20 ml Triäthylamin suspendiert und bei 0° unter Rühren mit 8 ml (0,084 Mol) Chlorameisensäureäthylester in 25 ml Methylenchlorid versetzt. Nach 1-stündigem Rühren bei 0° wird das Reaktionsgemisch mit Wasser gewaschen, und die organische Phase über Natriumsulfat getrocknet und unter vermindertem Druck zur Trockene eingedampft. Der Rückstand wird aus Methylenchlorid und Äthanol umkristallisiert, wobei man reines Diäthyl 6-diallylamino-s-triazin-2,4-dicarbamat-3-oxid vom Schmelzpunkt 123–125° erhält.

In analoger Weise erhält man

aus 5,0 g (0,0225 Mol) 2,4-Diamino-6-diallylamino-s-triazin-3-oxid und 5 ml (0,063 Mol) Chlorameisensäuremethylester das Dimethyl 6-diallylamino-s-triazin-2,4-dicarbamat-3-oxid, Smp. 158–160°;

aus 5,0 g (0,0225 Mol) 2,4-Diamino-6-diallylamino-s-triazin-3-oxid und 7 ml (0,053 Mol) Chlorameisensäureisobutylester das Diisobutyl 6-diallylamino-s-triazin-2,4-dicarbamat-3-oxid, Smp. 90–92°.

Beispiel 3

18 g (0,043 Mol) Diisobutyl 6-diallylamino-s-triazin-2,4-dicarbamat-3-oxid werden unter Rühren in 560 ml Methylenchlorid gelöst und mit 1800 ml Wasser und so viel konz. Natronlauge versetzt, bis der pH-Wert des Reaktionsgemisches 12,7 beträgt. Das Reaktionsgemisch wird dann während 75 Minuten gerührt, und die beiden Phasen werden danach getrennt. Die alkalische, wässrige Phase wird mit 3N Salzsäure auf pH 4 gestellt und dann mit Methylenchlorid dreimal extrahiert. Die organischen Extrakte werden über Natriumsulfat getrocknet und unter vermindertem Druck eingedampft. Der Rückstand wird aus Methylenchlorid und Diäthyläther umkristallisiert, wobei man reines Isobutyl 5-diallylamino -2-oxo-2H[1,2,4]oxadiazolo[2,3-a]-s-triazin-7-carbamat vom Schmelzpunkt 161–163° erhält.

Beispiel 4

4,0 g (0,016 Mol) 7-Amino-5-diallylamino-2H-[1,2,4]oxadiazolo[2,3-a]-s-triazin-2-on werden in 50 ml Methylenchlorid und 5 ml Triäthylamin suspendiert und auf 0° abgekühlt. Unter Rühren werden 5 ml (0,035 Mol) Chlorameisensäurebenzylester langsam zugetropft. Das Gemisch wird danach während 1 Stunde bei 0° weitergerührt, anschliessend mit Wasser versetzt und mit 2N Salzsäure auf pH 4 gestellt. Die wässrige Phase wird zweimal mit Methylenchlorid extrahiert, und die kombinierten organischen Extrakte werden über Natriumsulfat getrocknet und eingedampft. Der Rückstand wird aus Methylenchlorid und Diäthyläther umkristallisiert, wobei man reines Benzyl 5-diallylamino-2-oxo-2H-[1,2,4]oxadiazolo[2,3-a]-s-triazin-7-carbamat vom Schmelzpunkt 171–173° erhält.

In analoger Weise erhält man
aus 4,6 g (0,018 Mol) 7-Amino-5-diallylamino-2H-[1,2,4]oxadiazolo[2,3-a]-s-triazin-2-on und 4 ml (0,030 Mol) Chlorameisensäurebutylester reines Butyl 5-diallylamino-2-oxo-2H-[1,2,4]oxadiazolo [2,3-a]-s-triazin-7-carbamat, Smp. 135–137°;
aus 2,0 g (0,008 Mol) 7-Amino-5-diallylamino-2H-[1,2,4]oxadiazolo[2,3-a]-s-triazin-2-on und 3 ml (0,026 Mol) Chlorameisensäure-2-methoxyäthylester reines 2-Methoxyäthyl 5-diallylamino-2-oxo-2H-[1,2,4]oxadiazolo[2,3-a]-s-triazin-7-carbamat, Smp. 171–173°;
aus 4,0 g (0,016 Mol) 7-Amino-5-diallylamino-2H-[1,2,4]oxadiazolo[2,3-a]-s-triazin-2-on und 5 ml (0,047 Mol) Chlorameisensäureallylester reines Allyl 5-diallylamino-2-oxo-2H-[1,2,4]oxadiazolo [2,3-a]-s-triazin-7-carbamat, Smp. 173–175°.

Beispiel 5

4,5 g (0,018 Mol) 7-Amino-5-diallylamino-2H-[1,2,4]oxadiazolo[2,3-a]-s-triazin-2-on werden in 100 ml Methylenchlorid und 10 ml Triäthylamin suspendiert. Unter Rühren und Abkühlen werden 4,8 ml (0,036 Mol) Phenylacetylchlorid in 30 ml Methylenchlorid zugetropft. Das Gemisch wird danach bei Raumtemperatur während 70 Minuten gerührt, dann mit Wasser versetzt und mit 3N Salzsäure auf pH 4 gestellt. Die beiden Phasen werden getrennt, und die wässrige Phase nochmals mit Methylenchlorid extrahiert. Die kombinierten organischen Extrakte werden über Natriumsulfat getrocknet und unter vermindertem Druck zur Trockene eingedampft. Der Rückstand wird aus Methylenchlorid und Diäthyläther umkristallisiert, wobei man N-[5-Diallylamino-2-oxo-2H-[1,2,4]oxadiazolo[2,3-a]-s-triazin-7-yl]-2-phenylacetamid vom Schmelzpunkt 165–167° erhält.

In analoger Weise erhält man aus 4,5 g (0,018 Mol) 7-Amino-5-diallylamino-2H-[1,2,4]oxadiazolo[2,3-a]-s-triazin-2-on und 10,8 g (0,05 Mol) Homoveratrumsäurechlorid N-[5-Diallylamino-2-oxo -2H-[1,2,4]oxadiazolo[2,3-a]-s-triazin-7-yl]-2-(3,4-dimethoxyphenyl)acetamid, Smp. 139–141°.

Beispiel 6

4,5 g (0,018 Mol) 7-Amino-5-diallylamino-2H-[1,2,4]-oxadiazolo[2,3-a]-s-triazin-2-on werden in 100 ml Methylenchlorid und 10 ml Triäthylamin suspendiert, unter Rühren auf ca. 5° abgekühlt und mit 2,5 ml (0,024 Mol) 2,4-Dichlorbenzoylchlorid in 20 ml Methylenchlorid versetzt. Nach 1 Stunde bei 0° wird das Gemisch mit 100 ml Wasser verdünnt und mit Salzsäure auf pH 4 gestellt. Die beiden Phasen werden getrennt, und die wässrige Phase wird noch zweimal mit Methylenchlorid extrahiert. Die kombinierten organischen Extrakte werden über Natriumsulfat getrocknet und unter vermindertem Druck zur Trockene eingedampft. Der Rückstand wird unter Verwendung eines Gemisches von Chloroform und Methanol (99:1) als Eluierungsmittel über Kieselgel chromatographiert. Kristallisation des Rückstands aus Chloroform und Diäthyläther liefert 2,4-Dichlor-N-[5-diallylamino-2-oxo-2H-[1,2,4]oxadiazolo[2,3-a]-s-triazin-7-yl]benzamid vom Schmelzpunkt 163–164°.

In analoger Weise erhält man
aus 4,3 g (0,017 Mol) 7-Amino-5-diallylamino -2H-[1,2,4]oxadiazolo[2,3-a]-s-triazin-2-on und 4,8 g (0,023 Mol) 3,4-Dichlorbenzoylchlorid reines 3,4-Dichlor-N-[5-diallylamino-2-oxo-2H-[1,2,4]oxadiazolo-[2,3-a]-s-triazin-7-yl]benzamid, Smp. 190–191°;
aus 4,5 g (0,018 Mol) 7-Amino-5-diallylamino-2H-[1,2,4]oxadiazolo[2,3-a]-s-triazin-2-on und 7,2 g (0,036 Mol) 3,5-Dimethoxybenzoylchlorid reines N-[5-Diallylamino-2-oxo-2H-[1,2,4]oxadiazolo[2,3-a]-s-triazin-7-yl]-3,5-dimethoxybenzamid, Smp. 161–164°.

Beispiel 7

5,0 g (0,02 Mol) 7-Amino-5-diallylamino-2H-[1,2,4]oxadiazolo[2,3-a]-s-triazin-2-on werden mit 150 ml Chloroform, 10 ml Triäthylamin und 4,5 ml (0,032 Mol) 2,6-Dichlorbenzoylchlorid vermischt und während 7 Stunden zum Rückfluss erwärmt. Das Gemisch wird dann abgekühlt, mit Wasser verdünnt, mit Salzsäure auf pH 4 gestellt und mit Chloroform extrahiert. Die kombinierten organischen Extrakte werden über Natriumsulfat getrocknet und unter vermindertem Druck zur Trokkene eingedampft. Der Rückstand wird unter Verwendung eines Gemisches von Methylenchlorid und Methanol (99:1) als Eluierungsmittel über Kieselgel chromatographiert. Umkristallisation aus Isopropyläther und Methylenchlorid liefert reines 2,6-Dichlor-N-[5-diallylamino-2-oxo-2H-[1,2,4] oxadiazolo[2,3-a]-s-triazin-7-yl]-benzamid vom Schmelzpunkt 133–135°.

Beispiel 8

5,0 g (0,02 Mol) 7-Amino-5-diallylamino-2H-[1,2,4]oxadiazolo[2,3-a]-s-triazin-2-on werden in 7 ml Triäthylamin und 150 ml Methylenchlorid suspendiert. Unter Rühren und Abkühlen auf ca. 0° werden 3,4 ml (0,026 Mol) 4-Chlorbenzoylchlorid in 35 ml Methylenchlorid zugetropft. Das Gemisch wird anschliessend während 1 Stunde bei ca. 0° gerührt. Dann wird Wasser hinzugefügt und der pH mit verdünnter Salzsäurelösung auf 4 gestellt. Die beiden Phasen werden getrennt, und die wässrige Phase wird noch zweimal mit Methylenchlorid extrahiert. Die kombinierten organischen Extrakte werden über Natriumsulfat getrocknet und unter vermindertem Druck zur Trokkene eingedampft. Der Rückstand wird aus Methylenchlorid und Diäthyläther umkristallisiert, wobei man reines p-Chlor-N-[5-diallylamino-2-oxo-2H-[1,2,4]oxadiazolo[2,3-a]-s-triazin-7-yl]benzamid vom Schmelzpunkt 192–194° erhält.

In analoger Weise wurden die folgenden Verbindungen hergestellt:
aus 3,5 g (0,014 Mol) 7-Amino-5-diallylamino-2H-[1,2,4]oxadiazolo[2,3-a]-s-triazin-2-on und 2,2 ml (0,017 Mol) 2-Chlorbenzoylchlorid reines o-Chlor-N-[5-diallylamino-2-oxo-2H-[1,2,4]oxadiazolo[2,3-a]-s-triazin-7-yl]benzamid, Smp. 179–181°;

aus 4,5 g (0,018 Mol) 7-Amino-5-diallylamino-2H-[1,2,4]oxadiazolo[2,3-a]-s-triazin-2-on und 3,2 ml (0,025 Mol) 3-Chlorbenzoylchlorid reines m-Chlor-N-[5-diallylamino-2-oxo-2H-[1,2,4]oxadiazolo[2,3-a]-s-triazin-7-yl]benzamid, Smp. 172–173°;
aus 5,0 g (0,02 Mol) 7-Amino-5-diallylamino-2H-[1,2,4]oxadiazolo[2,3-a]-s-triazin-2-on und 5 g (0,025 Mol) 2-Acetylsalicylsäurechlorid reines o-[(5-Diallylamino-2-oxo-2H-[1,2,4]oxadiazolo[2,3-a]-s-triazin-7-yl) carbamoyl]phenylacetat, Smp. 159–161°;
aus 4,2 g (0,017 Mol) 7-Amino-5-diallylamino-2H-[1,2,4]oxadiazolo[2,3-a]-s-triazin-2-on und 3,1 g (0,019 Mol) p-Toluoylchlorid N-[5-Diallylamino-2-oxo-2H-[1,2,4]oxadiazolo[2,3-a]-s-triazin-7-yl]-p-toluamid, Smp. 151–153°;
aus 4,5 g (0,018 Mol) 7-Amino-5-diallylamino-2H-[1,2,4]oxadiazolo[2,3-a]-s-triazin-2-on und 3,4 g (0,022 Mol) m-Toluoylchlorid N-[5-Diallylamino-2-oxo-2H-[1,2,4]oxadiazolo[2,3-a]-s-triazin-7-yl]-m-toluamid, Smp. 159–160°;
aus 4,5 g (0,018 Mol) 7-Amino-5-diallylamino-2H-[1,2,4]oxadiazolo[2,3-a]-s-triazin-2-on und 4,0 g (0,023 Mol) Anissäurechlorid N-[5-Diallylamino-2-oxo-2H-[1,2,4]oxadiazolo[2,3-a]-s-triazin-7-yl]-p-anisamid, Smp. 185–186°;
aus 4,5 g (0,018 Mol) 7-Amino-5-diallylamino-2H-[1,2,4]oxadiazolo[2,3-a]-s-triazin-2-on und 3,0 g (0,019 Mol) 4-Cyanbenzoesäurechlorid p-Cyano-N-[5-Diallylamino-2-oxo-2H-[1,2,4]oxadiazolo[2,3-a]-s-triazin-7-yl]benzamid, Smp. 197–199°.

Beispiel 9

7,0 g (0,028 Mol) 7-Amino-5-diallylamino-2H-[1,2,4]oxadiazolo[2,3-a]-s-triazin-2-on werden in 250 ml Methylenchlorid und 10 ml Triäthylamin suspendiert, unter Rühren auf 0° abgekühlt und mit 4,5 ml (0,039 Mol) Benzoylchlorid in 35 ml Methylenchlorid versetzt. Das Gemisch wird anschliessend während 1 Stunde bei 0° gerührt, dann mit Wasser verdünnt und mit Salzsäure auf pH 4 gestellt. Die beiden Phasen werden getrennt, und die wässrige Phase noch zweimal mit Methylenchlorid extrahiert. Die organischen Extrakte werden kombiniert, über Magnesiumsulfat getrocknet und unter vermindertem Druck bei 20° eingedampft. Der Rückstand wird aus Methylenchlorid und Diäthyläther umkristallisiert, wobei man reines N-[5-Diallylamino-2-oxo-2H-[1,2,4]oxadiazolo[2,3-a]-s-triazin-7-yl]benzamid vom Schmelzpunkt 182–183° erhält.

Beispiel 10

10,4 g (0,042 Mol) 7-Amino-5-diallylamino-2H-[1,2,4]oxadiazolo[2,3-a]-s-triazin-2-on werden in 300 ml Methylenchlorid und 12 ml Triäthylamin suspendiert, unter Rühren auf 0° abgekühlt und mit 10 g (0,044 Mol) Benzoesäureanhydrid und 1,1 g 4-Dimethylaminopyridin versetzt. Das Gemisch wird anschliessend 45 Minuten bei 0° gerührt und dann mit Wasser gewaschen. Die organische Phase wird über Magnesiumsulfat getrocknet und eingeengt. Das Rohprodukt wird aus Methylenchlorid und Diäthyläther umkristallisiert, wobei man reines N-[5-Diallylamino-2-oxo-2H-[1,2,4]oxadiazolo[2,3-a]-s-triazin-7-yl]benzamid vom Schmelzpunkt 182–183° erhält.

Beispiel 11

3,3 g (0,027 Mol) Benzoesäure werden in 70 ml Methylenchlorid und 2,7 g (0,027 Mol) Triäthylamin gelöst. Die Lösung wird gerührt und bei 0° mit 3,5 g (0,027 Mol) Chlorameisensäureisobutylester in 25 ml Methylenchlorid versetzt. Das Gemisch wird dann zunächst 30 Minuten bei 0° und anschliessend 1 Stunde bei Raumtemperatur gerührt. Diese Lösung wird mit einer Suspension von 5 g (0,02 Mol) 7-Amino-5-diallylamino-2H-[1,2,4]oxadiazolo[2,3-a]-s-triazin-2-on in 200 ml Methylenchlorid, 12,5 ml Triäthylamin und 0,6 g 4-Dimethylaminopyridin versetzt. Das erhaltene Gemisch wird danach während 18 Stunden bei Raumtemperatur gerührt und dann mit 100 ml Wasser gewaschen. Die organische Phase wird über Magnesiumsulfat getrocknet und unter vermindertem Druck eingedampft. Der erhaltene Rückstand wird säulenchromatographisch gereinigt, wobei man N-[5-Diallylamino-2-oxo-2H-[1,2,4]oxadiazolo[2,3-a]-s-triazin-7-yl] benzamid vom Schmelzpunkt 182–183° erhält.

Beispiel 12

4,5 g (0,0128 Mol) N-[5-Diallylamino-2-oxo-2H-[1,2,4]oxadiazolo[2,3-a]-s-triazin-7-yl]benzamid werden in 100 ml Acetonitril gelöst und mit 12,8 ml (0,0128 Mol) IN Natronlauge versetzt. Das Gemisch wird unter vermindertem Druck eingeengt, und der erhaltene Rückstand aus Acetonitril/Wasser/Diäthyläther umkristallisiert, wobei man das reine, entsprechende Natriumsalz vom Schmelzpunkt 120–130° erhält.

Beispiel 13

3,5 g (0,01 Mol) N-[5-Diallylamino-2-oxo-2H-[1,2,4]oxadiazolo[2,3-a]-s-triazin-7-yl]benzamid werden in 50 ml Acetonitril gelöst und mit 1,8 g Dicyclohexylamin versetzt. Zugabe von Diäthyläther führt zur Ausfällung des reinen Dicyclohexylaminsalzes vom Schmelzpunkt 141–142°.

Beispiel 14

3,52 g (0,010 Mol) N-[5-Diallylamino-2-oxo-2H-[1,2,4]oxadiazolo[2,3-a]-s-triazin-7-yl]benzamid in 50 ml Acetonitril werden mit 1,3 g N-Äthyldiisopropylamin in 50 ml Acetonitril versetzt. Durch Zugabe von Diäthyläther fällt das entsprechende Äthyldiisopropylaminsalz vom Schmelzpunkt 106–108° aus.

Beispiel 15

3,52 g (0,01 Mol) N-[5-Diallylamino-2-oxo-2H-[1,2,4]oxadiazolo[2,3-a]-s-triazin-7-yl]benzamid in 50 ml Acetonitril werden mit 1,2 g tris-(Hydroxymethyl)aminomethan in 20 ml Acetonitril und 5 ml Wasser versetzt. Nach 30minütigem Rühren fällt das entsprechende Salz aus, Smp. 174–175°.

Beispiel 16

5 g (0,02 Mol) 7-Amino-5-diallylamino-2H-[1,2,4]oxadiazolo[2,3-a]-s-triazin-2-on werden in

80 ml Methylenchlorid und 5 ml Triäthylamin suspendiert. Bei 0° werden 2,2 ml (0,024 Mol) Propionsäurechlorid in 20 ml Methylenchlorid zugetropft. Das Gemisch wird während 90 Minuten gerührt, dann mit Wasser verdünnt, auf pH 4 gestellt und mit Methylenchlorid extrahiert. Die kombinierten organischen Extrakte werden über Magnesiumsulfat getrocknet und unter vermindertem Druck eingedampft. Der Rückstand wird mit Chloroform als Eluierungsmittel über Kieselgel chromatographiert. Umkristallisation aus Methylenchlorid und Diäthyläther liefert reines N-[5-Diallylamino-2-oxo-2H-[1,2,4]oxadiazolo[2,3-a]-s-triazin-7-yl]propionamid vom Schmelzpunkt 157–159°.

In analoger Weise erhält man

aus 4g (0,016 Mol) 7-Amino-5-diallylamino-2H-[1,2,4]oxadiazolo[2,3-a]-s-triazin-2-on und 5 ml (0,07 Mol) Acetylchlorid reines N-[5-Diallylamino-2-oxo-2H-[1,2,4]oxadiazolo[2,3-a]-s-triazin-7-yl]-acetamid, Smp. 164–165°;

aus 3,6 g (0,014 Mol) 7-Amino-5-diallylamino-2H-[1,2,4]oxadiazolo[2,3-a]-s-triazin-2-on und 5 ml (0,048 Mol) Buttersäurechlorid reines N-[5-Diallylamino-2-oxo-2H-[1,2,4]oxadiazolo[2,3-a]-s-triazin-7-yl]butyramid, Smp. 156–158°;

aus 5 g (0,02 Mol) 7-Amino-5-diallylamino-2H-[1,2,4]oxadiazolo[2,3-a]-s-triazin-2-on und 3,2 ml (0,026 Mol) Pivalinsäurechlorid reines N-[5-Diallylamino-2-oxo-2H-[1,2,4]oxadiazolo[2,3-a]-s-triazin-7-yl]pivalamid, Smp. 129–131°.

Beispiel 17

6g (0,024 Mol) 7-Amino-5-diallylamino-2H-[1,2,4]oxadiazolo[2,3-a]-s-triazin-2-on werden mit 60 ml abs. Pyridin und 8 ml (0,08 Mol) Essigsäureanhydrid während 18 Stunden bei Raumtemperatur gerührt. Die Lösungsmittel werden bei 40° abdestilliert, und der Rückstand in 220 ml Methylenchlorid gelöst. Die erhaltene Lösung wird mit 1N Salzsäure gewaschen, über Natriumsulfat getrocknet und unter vermindertem Druck zur Trokkene eingedampft. Der Rückstand wird aus Methylenchlorid und Diäthyläther umkristallisiert, wobei man N-[5-Diallylamino-2-oxo-2H-[1,2,4]oxadiazolo[2,3-a]-s-triazin-7-yl]acetamid vom Schmelzpunkt 164–165° erhält.

Beispiel 18

6,0 g (0,024 Mol) 7-Amino-5-diallylamino-2H-[1,2,4]oxadiazolo[2,3-a]-s-triazin-2-on werden in 200 ml Methylenchlorid und 10 ml Triäthylamin suspendiert. Das auf ca. 0° abgekühlte Gemisch wird unter Rühren mit 3 ml (0,030 Mol) Furan-2-carbonsäurechlorid in 50 ml Methylenchlorid versetzt. Nach 1-stündigem Rühren wird das Gemisch mit 100 ml Wasser verdünnt und mit Salzsäure auf pH 4 gestellt. Die beiden Phasen werden getrennt, die organische Phase wird über Magnesiumsulfat getrocknet und unter vermindertem Druck eingedampft. Der Rückstand wird aus Methylenchlorid und Diäthyläther umkristallisiert, wobei man reines N-[5-Diallylamino-2-oxo-2H-[1,2,4]oxadiazolo[2,3-a]-s-triazin-7-yl]-2-furamid vom Schmelzpunkt 159–162° erhält.

Beispiel 19

4,5 g (0,018 Mol) 7-Amino-5-diallylamino-2H-[1,2,4]oxadiazolo[2,3-a]-s-triazin-2-on werden in 100 ml Methylenchlorid und 15 ml Triäthylamin suspendiert. Bei 0° wird langsam eine Suspension von 4,5 g (0,025 Mol) Nicotinsäurechlorid-hydrochlorid in 50 ml Methylenchlorid zugetropft. Die dunkle Lösung wird während 2 Stunden bei 0° gerührt, dann zweimal mit Wasser gewaschen und über Natriumsulfat getrocknet. Das Lösungsmittel wird unter vermindertem Druck abdestilliert, und der Rückstand über Kieselgel chromatographiert. Das so erhaltene Rohprodukt wird in Methylenchlorid mit Salzsäure in Dioxan versetzt, wobei man reines N-[5-Diallylamino-2-oxo-2H-[1,2,4]oxadiazolo[2,3-a]-s-triazin-7-yl]nicotinamid-dihydrochlorid vom Schmelzpunkt 135–140° erhält.

In analoger Weise erhält man

aus 4,5 g (0,018 Mol) 7-Amino-5-diallylamino-2H-[1,2,4]oxadiazolo[2,3-a]-s-triazin-2-on und 4,5 g (0,025 Mol) Isonicotinsäurechlorid-hydrochlorid N-[5-Diallylamino-2-oxo-2H-[1,2,4]oxadiazolo[2,3-a]-s-triazin-7-yl]isonicotinamid-dihydrochlorid, Smp. 130–140°;

aus 3,6 g (0,014 Mol) 7-Amino-5-diallylamino-2H-[1,2,4]oxadiazolo[2,3-a]-s-triazin-2-on und 4,3 g (0,024 Mol) 2-Picolinsäurechlorid-hydrochlorid reines N-[5-Diallylamino-2-oxo-2H-[1,2,4]oxadiazolo[2,3-a]-s-triazin-7-yl]-2-pyridincarboxamid, Smp. 213–215°;

aus 4,5 g (0,018 Mol) 7-Amino-5-diallylamino-2H-[1,2,4]oxadiazolo[2,3-a]-s-triazin-2-on und 2,4 ml (0,0225 Mol) Thiophen-2-carbonsäurechlorid reines N-[5-Diallylamino-2-oxo-2H-[1,2,4]oxadiazolo[2,3-a]-s-triazin-7-yl]-2-thiophencarboxamid, Smp. 179–181°.

Beispiel 20

5,0 g (0,02 Mol) 7-Amino-5-diallylamino-2H-[1,2,4]oxadiazolo[2,3-a]-s-triazin-2-on werden in 100 ml Methylenchlorid und 10 ml Triäthylamin suspendiert. Unter Rühren und Abkühlen auf ca. 5° werden 2,5 g (0,022 Mol) Methoxyessigsäurechlorid in 20 ml Methylenchlorid zugetropft. Nach 1 Stunde bei ca. 5° wird das Gemisch mit Wasser versetzt und mit 3N Salzsäure auf pH 4 gestellt. Die beiden Phasen werden getrennt, und die wässrige Phase nochmals mit Methylenchlorid extrahiert. Die kombinierten organischen Extrakte werden über Natriumsulfat getrocknet und unter vermindertem Druck eingedampft. Der Rückstand wird aus Methylenchlorid und Diäthyläther umkristallisiert, wobei man N-[5-Diallylamino-2-oxo-2H-[1,2,4]oxadiazolo[2,3-a]-s-triazin-7-yl]-2-methoxyacetamid vom Schmelzpunkt 135–136° erhält.

In analoger Weise erhält man

aus 7,5 g (0,03 Mol) 7-Amino-5-diallylamino-2H-[1,2,4]oxadiazolo[2,3-a]-s-triazin-2-on und 2,9 ml (0,036 Mol) Chloracetylchlorid 2-Chlor-N-[5-diallylamino-2-oxo-2H-[1,2,4]oxadiazolo[2,3-a]-s-triazin-7-yl]acetamid, Smp. 159–162°;

aus 5,2 g (0,021 Mol) 7-Amino-5-diallylamino-2H-[1,2,4]oxadiazolo[2,3-a]-s-triazin-2-on und 2,6 g (0,021 Mol) Äthoxyessigsäurechlorid 2-Äthoxy-N-[5-diallylamino-2-oxo-2H-[1,2,4]oxadiazolo[2,3-a]-s-triazin-7-yl]acetamid, Smp. 111–113°;
aus 4,8 g (0,019 Mol) 7-Amino-5-diallylamino-2H-[1,2,4]oxadiazolo[2,3-a]-s-triazin-2-on und 5,2 g (0,030 Mol) Phenoxyessigsäurechlorid N-[5-Diallylamino-2-oxo-2H-[1,2,4]oxadiazolo[2,3-a]-s-triazin-7-yl]-2-phenoxyacetamid, Smp. 164–166°.

Beispiel 21

6,5 g (0,026 Mol) 7-Amino-5-diallylamino-2H-[1,2,4]oxadiazolo[2,3-a]-s-triazin-2-on werden in 120 ml Methylenchlorid und 8 ml Triäthylamin suspendiert. Bei 0° werden unter Rühren 3 ml (0,037 Mol)Acrylsäurechlorid in 35ml Methylenchlorid zugetropft. Das Reaktionsgemisch wird dann während 2 Stunden gerührt, danach mit 100 ml Wasser verdünnt und mit 2N Salzsäure auf pH 4 gestellt. Die beiden Phasen werden getrennt, und die wässrige Phase nochmals mit Methylenchlorid extrahiert. Die kombinierten organischen Extrakte werden über Natriumsulfat getrocknet und unter vermindertem Druck zur Trokkene eingedampft. Der Rückstand wird mit Methylenchlorid und Methanol als Eluierungsmittel über Kieselgel chromatographiert, und die gewünschte Substanz aus Methylenchlorid und Diäthyläther umkristallisiert, wobei man reines N-[5-Diallylamino-2-oxo-2H-[1,2,4]oxadiazolo[2,3-a]-s-triazin-7-yl]acrylamid vom Schmelzpunkt 157–161° erhält.

In analoger Weise erhält man
aus 5 g (0,02 Mol) 7-Amino-5-diallylamino-2H-[1,2,4]oxadiazolo[2,3-a]-s-triazin-2-on und 2,3 ml (0,024 Mol) Methacrylsäurechlorid reines N-[5-Diallylamino-2-oxo-2H-[1,2,4]oxadiazolo[2,3-a]-s-triazin-7-yl]-2-methylacrylamid, Smp. 110–112°;
aus 4,5 g (0,018 Mol) 7-Amino-5-diallylamino-2H-[1,2,4]oxadiazolo[2,3-a]-s-triazin-2-on und 2,25 ml (0,023 Mol) Crotonsäurechlorid N-[5-Diallylamino-2-oxo-2H-[1,2,4]oxadiazolo[2,3-a]-s-triazin-7-yl]-crotonamid, Smp. 154–157°;
aus 4,5 g (0,018 Mol) 7-Amino-5-diallylamino-2H-[1,2,4]oxadiazolo[2,3-a]-s-triazin-2-on und 4,5 g (0,028 Mol) Zimtsäurechlorid N-[5-Diallylamino-2-oxo-2H-[1,2,4]oxadiazolo[2,3-a]-s-triazin-7-yl]zimtsäureamid, Smp. 215–216°;
aus 5,2 g (0,021 Mol) 7-Amino-5-diallylamino-2H-[1,2,4]oxadiazolo[2,3-a]-s-triazin-2-on und 4,0 g (0,025 Mol) 3-(2-Furyl)acrylsäurechlorid N-[5-Diallylamino-2-oxo-2H-[1,2,4]oxadiazolo[2,3-a]-s-triazin-7-yl]-2-furanacrylamid, Smp. 224–226°.

Beispiel 22

4,5 g (0,018 Mol) 7-Amino-5-diallylamino-2H-[1,2,4]oxadiazolo[2,3-a]-s-triazin-2-on werden mit 10 ml Triäthylamin in 150 ml Methylenchlorid suspendiert. Unter Rühren wird das Gemisch abgekühlt und mit 3,6 ml (0,026 Mol) Glutarsäuremonomethylesterchlorid versetzt. Nach 45minütigem Rühren wird das Gemisch mit Wasser verdünnt, mit Salzsäure auf pH 4,5 gestellt und mit Methylenchlorid extrahiert. Die kombinierten organischen Extrakte werden über Natriumsulfat getrocknet und unter vermindertem Druck zur Trokkene eingedampft. Das Rohprodukt wird mit einem Gemisch von Chloroform und Methanol (99:1) als Eluierungsmittel über Kieselgel chromatographiert. Kristallisation des Rückstandes aus Methylenchlorid und Diäthyläther liefert reines Methyl N-[5-diallylamino-2-oxo-2H-[1,2,4]oxadiazolo[2,3-a]-s-triazin-7-yl]glutaramat vom Schmelzpunkt 164–165°.

In analoger Weise erhält man
aus 4,5 g (0,018 Mol) 7-Amino-5-diallylamino-2H-[1,2,4]oxadiazolo[2,3-a]-s-triazin-2-on und 2,15 ml (0,019 Mol) Malonsäuremonomethylesterchlorid reines Methyl N-[5-diallylamino-2-oxo-2H-[1,2,4]oxadiazolo[2,3-a]-s-triazin-7-yl]malonamat, Smp. 138–140°;
aus 4,5 g (0,018 Mol) 7-Amino-5-diallylamino-2H-[1,2,4]oxadiazolo[2,3-a]-s-triazin-2-on und 3,6 ml (0,028 Mol) Bernsteinsäuremonomethylesterchlorid reines Methyl N-[5-diallylamino-2-oxo-2H-[1,2,4]oxadiazolo[2,3-a]-s-triazin-7-yl]succinamat, Smp. 129–131°.

Beispiel 23

3,0 g (0,012 Mol) 7-Amino-5-diallylamino-2H-[1,2,4]oxadiazolo[2,3-a]-s-triazin-2-on werden in 100 ml Aceton gelöst und mit 4 ml (0,045 Mol) Chloressigsäuremethylester und 4 g Kaliumcarbonat versetzt. Das Gemisch wird während 21 Stunden kräftig gerührt und dann unter vermindertem Druck zur Trockene eingedampft. Der Rückstand wird in Methylenchlorid und Wasser gelöst und zweimal mit Methylenchlorid extrahiert. Die kombinierten organischen Extrakte werden über Kaliumcarbonat getrocknet und unter vermindertem Druck eingedampft. Das Rohprodukt wird mit einem Gemisch von Methylenchlorid und Methanol (99:1) als Eluierungsmittel über Kieselgel chromatographiert, wobei man reinen N-[5-Diallylamino-2-oxo-2H-[1,2,4]oxadiazolo[2,3-a]-s-triazin-7-yl] glycinmethylester erhält, der aus Aceton und Petroläther umkristallisiert wird, Smp. 173–174°.

In analoger Weise erhält man
aus 5 g (0,02 Mol) 7-Amino-5-diallylamino-2H-[1,2,4]oxadiazolo[2,3-a]-s-triazin-2-on und 7,8 ml (0,06 Mol) 2-Brompropionsäureäthylester reinen N-[5-Diallylamino-2-oxo-2H-[1,2,4]oxadiazolo[2,3-a]-s-triazin-7-yl]-DL-alaninäthylester, Smp. 135–137°;
aus 5 g (0,02 Mol) 7-Amino-5-diallylamino-2H-[1,2,4]oxadiazolo[2,3-a]-s-triazin-2-on und 8,9 ml (0,06 Mol) 2-Brombuttersäureäthylester reines Äthyl rac-2-[(5-diallylamino-2-oxo-2H-[1,2,4]oxadiazolo[2,3-a]-s-triazin-7-yl)amino]butyrat, Smp. 94–95°.

Beispiel 24

4,8 g (0,014 Mol) Diäthyl 6-[3,6-dihydro-1(2H)-pyridyl]-s-triazin-2,4-dicarbamat-3-oxid werden mit 100 ml abs. Dimethylformamid vermischt und während 30 Minuten bei 140° gerührt. Das Lösungsmittel wird dann unter vermindertem Druck eingedampft, und der Rückstand aus Methylenchlorid und Diäthyläther umkristallisiert, wobei man reines Äthyl 5-[3,6-dihydro-1(2H)-pyridyl]-2-oxo-2H-[1,2,4]oxadiazolo[2,3-a]-s-triazin-7-carbamat vom Schmelzpunkt 220–225° (Zers.) erhält.

In analoger Weise wurden die folgenden Verbindungen hergestellt:
aus 4,8 g (0,015 Mol) Dimethyl 6-[3,6-dihydro-1(2H)-pyridyl]-s-triazin-2,4-dicarbamat-3-oxid das Methyl 5-[3,6-dihydro-1(2H)-pyridyl]-2-oxo-2H-[1,2,4]oxadiazolo[2,3-a]-s-triazin-7-carbamat, Smp. 217–220°;
aus 6,5 g (0,016 Mol) Diisobutyl 6-[3,6-dihydro-1(2H)-pyridyl]-s-triazin-2,4-dicarbamat-3-oxid das Isobutyl 5-[3,6-dihydro-1(2H)-pyridyl]-2-oxo-2H-[1,2,4]oxadiazolo[2,3-a]-s-triazin-7-carbamat, Smp. 210–215° (Zers.).

Das als Ausgangsmaterial verwendete Diäthyl 6-[3,6-dihydro-1(2H)-pyridyl]-s-triazin-2,4-dicarbamat-3-oxid kann wie folgt hergestellt werden:

300 g (2,06 Mol) 2-Chlor-4,6-diamino-s-triazin, 180 g (2,16 Mol) 1,2,5,6-Tetrahydropyridin und 170g (2,02 Mol) Natriumbicarbonat werden in 1700 ml n-Butanol vermischt und während 18 Stunden zum Rückfluss erhitzt. Das Reaktionsgemisch wird abgekühlt und filtriert. Der Rückstand wird dreimal mit heissem Chloroform und Äthanol digeriert und filtriert. Die Filtrate werden kombiniert und eingeengt, wobei man reines 2,4-Diamino-6-[3,6-dihydro-1(2H)-pyridyl]-s-triazin vom Schmelzpunkt 200–202° erhält.

191 g (1 Mol) m-Chlorperbenzoesäure (90%ig) werden in 800 ml Dimethoxyäthan gelöst und auf −5° abgekühlt. Dazu werden 103 g (0,495 Mol) 2,4-Diamino-6-[3,6-dihydro-1(2H)-pyridyl]-s-triazin in fester Form innerhalb von 2,5 Stunden hinzugefügt. Man lässt auf Raumtemperatur aufwärmen und rührt das Gemisch über Nacht. Das Lösungsmittel wird dann unter vermindertem Druck bei 20° eingeengt, und 200 ml Wasser werden hinzugefügt. Der pH-Wert wird dann mit Natronlauge auf 10 gestellt, und die Lösung kontinuierlich mit Chloroform während 20 Stunden extrahiert. Das Lösungsmittel wird dann abgedampft, und das Rohprodukt wird über Kieselgel chromatographiert, wobei man reines 2,4-Diamino-6-[3,6-dihydro-1(2H)-pyridyl]-s-triazin-3-oxid vom Schmelzpunkt 261–265° (Zers.) erhält. Diese Verbindung kann auch wie folgt hergestellt werden:

40 g (0,335 Mol) 1,2,5,6-Tetrahydropyridin-hydrochlorid und 30 g (0,337 Mol) Natriumdicyanamid werden in 400 ml n-Butanol und 30 ml Wasser suspendiert und über Nacht zum Rückfluss erhitzt. Das Gemisch wird abgekühlt, filtriert, und das Filtrat eingedampft. Der Rückstand wird in 100 ml Wasser digeriert, der Festkörper abfiltriert und aus o-Xylol umkristallisiert, wobei man reines 1-[1,2,5,6-Tetrahydropyridin]-3-cyanoguanidin vom Schmelzpunkt 131–133° erhält.

60 g (0,4 Mol) 1-(1,2,5,6-Tetrahydropyridin)-3-cyanoguanidin werden mit 1000 ml absolutem Tetrahydrofuran und 46 g (0,41 Mol) Kalium-tert.-butylat vermischt und 5 Minuten zum Rückfluss erwärmt. Das Gemisch wird auf 5° abgekühlt und mit 43 g (0,406 Mol) Bromcyan versetzt, wobei die Temperatur auf 30° ansteigt. Man gibt dann 46 g (0,41 Mol) Kalium-tert.-butylat zu und rührt das Gemisch während 15 Minuten bei Raumtemperatur. Das Lösungsmittel wird dann unter vermindertem Druck abgedampft, und der Rückstand in 500 ml Wasser gelöst und mit 28 g (0,403 Mol) Hydroxylamin-hydrochlorid versetzt. Das Gemisch wird dann 45 Minuten zum Rückfluss erwärmt. Nach Abkühlen wird die Lösung mit Salzsäure sauer gestellt und zweimal mit Methylenchlorid extrahiert. Die wässrige Phase wird dann mit Natronlauge auf pH 9 gestellt und über Nacht mit Chloroform extrahiert. Nach Eindampfen des Lösungsmittels isoliert man reines 2,4-Diamino-6-[3,6-dihydro-1(2H)-pyridyl]-s-triazin-3-oxid vom Schmelzpunkt 260–265° (Zers.).

10 g (0,048 Mol) 2,4-Diamino-6-[3,6-dihydro-1(2H)-pyridyl]-s-triazin-3-oxid werden in 350 ml Methylenchlorid und 40 ml Triäthylamin suspendiert und auf 5° abgekühlt. Unter Rühren werden langsam 10 ml (0,105 Mol) Chlorameisensäureäthylester in 60 ml Methylenchlorid zugetropft. Das Gemisch wird 1 Stunde bei 5° und 1 Stunde bei Raumtemperatur gerührt, mit Wasser gewaschen, und die wässrige Phase nochmals mit Methylenchlorid extrahiert. Die kombinierten organischen Extrakte werden über Natriumsulfat getrocknet und unter vermindertem Druck eingedampft. Der Rückstand wird aus Methylenchlorid und Diäthyläther umkristallisiert, wobei man reines Diäthyl 6-[3,6-dihydro-1(2H)-pyridyl]-s-triazin-2,4-dicarbamat-3-oxid vom Schmelzpunkt 143–148° (Zers.) erhält.

In analoger Weise wurden die folgenden, ebenfalls als Ausgangsstoffe verwendeten Verbindungen hergestellt:
aus 6,2 g (0,030 Mol) 2,4-Diamino-6-[3,6-dihydro-1(2H)-pyridyl]-s-triazin-3-oxid und 6 ml (0,076 Mol) Chloroameisensäuremethylester das Dimethyl 6-[3,6-dihydro-1(2H)-pyridyl]-s-triazin-2,4-dicarbamat-3-oxid, Smp. 190–200° (Zers.);
aus 5,8 g (0,028 Mol) 2,4-Diamino-6-[3,6-dihydro-1(2H)-pyridyl]-s-triazin-3-oxid und 7 ml (0,0533 Mol) Chlorameisensäureisobutylester das Diisobutyl 6-[3,6-dihydro-1(2H)-pyridyl]-s-triazin-2,4-dicarbamat-3-oxid, Smp. 133–135°.

Beispiel 25

8 g (0,019 Mol) Diisobutyl 6-[3,6-dihydro-1(2H)-pyridyl]-s-triazin-2,4-dicarbamat-3-oxid werden in 300 ml Methylenchlorid und 1000 ml Wasser gelöst und mit konz. Natronlauge auf pH 12,7 gestellt. Das Gemisch wird 75 Minuten gerührt, die organische Phase wird abgetrennt, und die wässrige Phase mit 3N Salzsäurelösung auf pH 4 gestellt. Die so erhaltene saure, wässrige Lösung wird mit Methylenchlorid extrahiert. Die organischen Extrakte werden über Natriumsulfat getrocknet und eingedampft, wobei man reines Isobutyl 5-[3,6-dihydro-1(2H)-pyridyl]-2-oxo-2H-[1,2,4]oxadiazolo[2,3-a]-s-triazin-7-carbamat vom Schmelzpunkt 210–215° erhält.

In analoger Weise erhält man
aus Diäthyl 6-[3,6-dihydro-1(2H)-pyridyl]-s-triazin-2,4-dicarbamat-3-oxid das Äthyl 5-[3,6-dihydro-1(2H)-pyridyl]-2-oxo-2H-[1,2,4]oxadiazolo[2,3-a]-s-triazin-7-carbamat, Smp. 220–225°;
aus Dimethyl 6-[3,6-dihydro-1(2H)-pyridyl]-s-triazin-2,4-dicarbamat-3-oxid das Methyl 5-[3,6-dihydro-1(2H)-pyridyl]-2-oxo-2H-[1,2,4]oxadiazolo[2,3-a]-s-triazin-7-carbamat, Smp. 217–220°.

Beispiel 26

9,5 g (0,046 Mol) 2,4-Diamino-6-[3,6-dihydro-1(2H)-pyridyl]-s-triazin-3-oxid werden in 250 ml Methylenchlorid suspendiert und unter Rühren auf 5° abgekühlt. Bei dieser Temperatur werden gleichzeitig 25 ml (0,048 Mol) 20%iges Phosgen in Toluol und 10%ige Natronlauge innerhalb von 30 Minuten so zugetropft, dass der pH-Wert bei 7–8 gehalten wird. Das Gemisch wird noch 1 Stunde gerührt, und die beiden Phasen getrennt. Die wässrige Phase wird noch zweimal mit Methylenchlorid und Methanol extrahiert, und die organischen Extrakte über Magnesiumsulfat getrocknet. Durch Abdampfen des Lösungsmittels erhält man reines 7-Amino-5-[3,6-dihydro-1(2H)-pyridyl]-2-oxo-2H-[1,2,4]oxadiazolo[2,3-a]-s-triazin-2-on vom Schmelzpunkt 220–235° (Zers.).

Beispiel 27

6,9 g (0,029 Mol) 7-Amino-5-[3,6-dihydro-1(2H)-pyridyl]-2-oxo-2H-[1,2,4]oxadiazolo[2,3-a]-s-triazin-2-on werden in 200 ml Methylenchlorid und 8 ml Triäthylamin suspendiert, und bei 0° werden unter Rühren 5,2 ml (0,055 Mol) Chlorameisensäureäthylester in 70 ml Methylenchlorid zugetropft. Nach 1 Stunde bei 0° wird der pH-Wert mit 1N Salzsäure auf 4,5 gestellt, und die beiden Phasen getrennt. Die organische Phase wird über Natriumsulfat getrocknet, und das Lösungsmittel abdestilliert. Der Rückstand wird aus Methylenchlorid und Diäthyläther umkristallisiert, wobei man reines Äthyl 5-[3,6-dihydro-1(2H)-pyridyl]-2-oxo-2H-[1,2,4]oxadiazolo[2,3-a]-s-triazin-7-carbamat vom Schmelzpunkt 218–225° (Zers.) erhält.

Beispiel 28

5,0 g (0,02 Mol) 7-Amino-5-diallylamino-2H-[1,2,4]oxadiazolo[2,3-a]-s-triazin-2-on werden in 100 ml Methylenchlorid und 10 ml Triäthylamin suspendiert. Unter Rühren und Abkühlen auf ca. 0° werden 5,8 ml (0,04 Mol) Thiophen-2-acetylchlorid in 20 ml Methylenchlorid zugetropft. Das Gemisch wird danach während 4 Stunden bei ca. 5° weitergerührt, anschliessend mit Wasser versetzt und mit 3N Salzsäure auf pH 3,7 gestellt. Die beiden Phasen werden getrennt, und die wässrige Phase nochmals mit Methylenchlorid extrahiert. Die kombinierten organischen Phasen werden über Natriumsulfat getrocknet und unter vermindertem Druck eingedampft. Der Rückstand wird mit Methylenchlorid und Methanol an Kieselgel chromatographiert. Umkristallisation aus Methylenchlorid/Diäthyläther liefert reines N-(5-Diallylamino-2-oxo-2H-[1,2,4]oxadiazolo[2,3-a]-s-triazin-7-yl)-2-thiophenacetamid vom Schmelzpunkt 140–142°.

Beispiel 29

4,5 g (0,018 Mol) 7-Amino-5-diallylamino-2H-[1,2,4]oxadiazolo[2,3-a]-s-triazin-2-on werden in 100 ml Triäthylamin und 0,5 g 4-Dimethylaminopyridin suspendiert. Unter Rühren und Abkühlen auf ca. 0° werden 11,6 g (0,07 Mol) Thiophen-3-acetylchlorid in 40 ml Methylenchlorid zugetropft. Das Gemisch wird danach während 1 Stunde bei ca. 5° weitergerührt, anschliessend mit Wasser versetzt und mit 3N Salzsäure auf pH 4 gestellt. Die beiden Phasen werden getrennt, und die wässrige Phase nochmals mit Methylenchlorid extrahiert. Die kombinierten organischen Extrakte werden über Magnesiumsulfat getrocknet und unter vermindertem Druck eingedampft. Der Rückstand wird aus wenig Methylenchlorid und viel Diäthyläther umkristallisiert, wobei man reines N-(5-Diallylamino-2-oxo-2H-[1,2,4]oxadiazolo[2,3-a]-s-triazin-7-yl)-3-thiophenacetamid vom Schmelzpunkt 159–161° erhält.

Beispiel 30

15 g (0,06 Mol) 7-Amino-5-diallylamino-2H-[1,2,4]oxadiazolo[2,3-a]-s-triazin-2-on werden in 300 ml Methylenchlorid, 30 ml Triäthylamin und 0,9 g 4-Dimethylaminopyridin suspendiert. Unter Rühren und Abkühlen auf ca. 5° werden 10,66 g (0,09 Mol) 3,3-Dimethylacrylsäurechlorid in 100 ml Methylenchlorid zugetropft. Das Reaktionsgemisch wird danach während 1 Stunde bei 0° weitergerührt, mit Wasser versetzt und mit 3N Salzsäure auf pH 4 gestellt. Die beiden Phasen werden getrennt, und die wässrige Phase nochmals mit Methylenchlorid extrahiert. Die kombinierten organischen Extrakte werden über Magnesiumsulfat getrocknet und unter vermindertem Druck eingedampft. Der Rückstand wird aus Methylenchlorid/Diäthyläther umkristallisiert, wobei man reines N-(5-Diallylamino-2-oxo-2H-[1,2,4]oxadiazolo[2,3-a]-s-triazin-7-yl)-3-methylcrotonamid vom Schmelzpunkt 165–166° erhält.

Beispiel 31

22 g (0,089 Mol) 7-Amino-5-diallylamino-2H-[1,2,4]oxadiazolo[2,3-a]-s-triazin-2-on werden in 500 ml Methylenchlorid und 25 ml Triäthylamin suspendiert. Unter Rühren bei Raumtemperatur werden 19,6 g (0,1 Mol) 3,3-Dimethylacrylsäureanhydrid in 400 ml Methylenchlorid zugegeben. Das Gemisch wird danach während 90 Minuten bei Raumtemperatur weitergerührt. Dann wird das Lösungsmittel mit Wasser und anschliessend mit 3N Salzsäure gewaschen. Die erhaltene organische Phase wird über Natriumsulfat getrocknet und unter vermindertem Druck eingeengt. Der Rückstand wird aus Methylenchlorid/Diäthyläther umkristallisiert, wobei man reines N-(5-Diallylamino-2-oxo-2H-[1,2,4]oxadiazolo[2,3-a]-s-triazin-7-yl]-3-methylcrotonamid vom Schmelzpunkt 165–166° erhält.

Beispiel A

Herstellung von Tabletten folgender Zusammensetzung:

| | | |
|---|---|---|
| I | N-(5-Diallylamino-2-oxo-2H-[1,2,4]oxadiazolo[2,3-a]-s-triazin-7-yl)benzamid mikronisiert | 50,0 mg |
| | Milchzucker pulv. | 91,8 mg |
| | Maisstärke weiss | 75,0 mg |
| II | Dioctylnatriumsulfosuccinat | 0,2 mg |
| | Maisstärke weiss | 8,0 mg |
| | Wasser | q.s. |

| | | |
|---|---|---|
| III | Maisstärke weiss | 20,0 mg |
| IV | Talk | 4,0 mg |
| | Magnesiumstearat | 1,0 mg |
| | | 250,0 mg |

Die Stoffe der Phase I werden gesiebt und gemischt. Diese Mischung wird mit dem Maisstärkekleister II befeuchtet und geknetet. Die feuchte Masse wird granuliert, getrocknet und auf eine geeignete Korngrösse zugerüstet. Phase III wird beigemischt. Diese Mischung wird noch während kurzer Zeit mit Phase IV gemischt.

Die pressfertige Mischung wird zu Tabletten à 250 mg von 10 mm Durchmesser mit Bruchrille verpresst.

Beispiel B

Herstellung von Tabletten folgender Zusammensetzung:

| | | |
|---|---|---|
| I | N-(5-Diallylamino-2-oxo-2H-[1,2,4]oxadiazolo[2,3-a]-s-triazin-7-yl)benzamid mikronisiert | 100,0 mg |
| | Milchzucker pulv. | 66,8 mg |
| | Maisstärke weiss | 50,0 mg |
| II | Dioctylnatriumsulfosuccinat | 0,2 mg |
| | Maisstärke weiss | 8,0 mg |
| | Wasser | q.s. |
| III | Maisstärke weiss | 20,0 mg |
| IV | Talk | 4,0 mg |
| | Magnesiumstearat | 1,0 mg |
| | | 250,0 mg |

Die Stoffe der Phase I werden gesiebt und gemischt. Diese Mischung wird mit dem Maisstärkekleister II befeuchtet und geknetet. Die feuchte Masse wird granuliert, getrocknet und auf eine geeignete Korngrösse zugerüstet. Phase III wird beigemischt. Diese Mischung wird noch während kurzer Zeit mit Phase IV gemischt.

Die pressfertige Mischung wird zu Tabletten à 250 mg von 10 mm Durchmesser mit Bruchrille verpresst.

Beispiel C

Herstellung von Tabletten folgender Zusammensetzung:

| | | |
|---|---|---|
| I | N-(5-Diallylamino-2-oxo-2H-[1,2,4]oxadiazolo[2,3-a]-s-triazin-7-yl)benzamid mikronisiert | 250,0 mg |
| | Milchzucker pulv. | 83,6 mg |
| | Maisstärke weiss | 100,0 mg |
| II | Dioctylnatriumsulfosuccinat | 0,4 mg |
| | Maisstärke weiss | 16,0 mg |
| | Wasser | q.s. |
| III | Maisstärke weiss | 40,0 mg |
| IV | Talk | 8,0 mg |
| | Magnesiumstearat | 2,0 mg |
| | | 500,0 mg |

Die Stoffe der Phase I werden gesiebt und gemischt. Diese Mischung wird mit dem Maisstärkekleister II befeuchtet und geknetet. Die feuchte Masse wird granuliert, getrocknet und auf eine geeignete Korngrösse zugerüstet. Phase III wird beigemischt. Diese Mischung wird noch während kurzer Zeit mit Phase IV gemischt.

Die pressfertige Mischung wird zu Tabletten à 500 mg von 12 mm Durchmesser mit Bruchrille verpresst.

Beispiel D

Herstellung von Injektionspräparaten folgender Zusammensetzung:

| | |
|---|---|
| N-[5-Diallylamino-2-oxo-2H-[1,2,4]oxadiazolo[2,3-a]-s-triazin-7-yl]-benzamid | 50–250 mg |
| Mannit | 180– 80 mg |
| 2N Natronlauge ad pH 12,3 | q.s. |
| 1N Salzsäure ad pH 9,3 | q.s. |
| Wasser zu Injektionszwecken ad | 5,0 ml |

Zur Herstellung der spritzfertigen Lösung ist der Inhalt einer Trockenampulle mit 4,9 ml Wasser aufzulösen. 5,0 ml dieser Lösung enthalten 50–250 mg des Wirkstoffes.

Beispiel E

Wenn man nach den in den Beispielen A bis D beschriebenen Verfahren arbeitet, können aus den folgenden ebenfalls bevorzugten Verbindungen und ihren pharmazeutisch verwendbaren Salzen Tabletten bzw. Injektionspräparate hergestellt werden:

N-(5-Diallylamino-2-oxo-2H-[1,2,4]oxadiazolo[2,3-a]-s-triazin-7-yl)-3-methylcrotonamid.

N-(5-Diallylamino-2-oxo-2H-[1,2,4]oxadiazolo[2,3-a]-s-triazin-7-yl)-2-methylacrylamid.

**Patentansprüche für die Vertragsstaaten BE, CH, DE, FR, GB, IT, LU, NL, SE**

1. Oxadiazolotriazinderivate der allgemeinen Formel

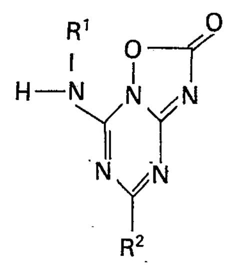

I

worin $R^1$ Wasserstoff oder eine der Gruppen

$-\overset{\overset{O}{\|}}{C}-R^3$, $-COOR^4$ und $-CH(R^5)COOR^6$ bedeutet und $R^2$ Diallylamino oder 1,2,5,6-Tetrahydropyridin-1-yl, $R^3$ Alkyl, Haloalkyl, Alkoxyalkyl, Aryloxyalkyl, Arylalkyloxyalkyl, Arylalkyl, Alkoxycarbonylalkyl, Aryl, die Gruppe $-C(R^7)=C(R^8)(R^9)$, Alkoxycarbonylalkylcarbonyl oder gegebenenfalls über eine Methylengruppe gebundenes Pyridyl, Furyl oder Thienyl, $R^4$ Alkyl, Alkoxyalkyl, Arylalkyl, Aryl oder Allyl, $R^5$ Wasserstoff oder Alkyl, $R^6$ Alkyl, $R^7$ und $R^8$ je Wasserstoff, Alkyl, Aryl, Arylalkyl, Pyridyl, Furyl

oder Thienyl und $R^9$ Wasserstoff oder Methyl bedeuten, wobei die Alkyl- und Alkoxyreste 1–8 Kohlenstoffatome aufweisen und der Arylrest – allein oder in Kombination – einen ein- oder zweikernigen aromatischen Rest mit bis zu 12 Kohlenstoffatomen bedeutet, in welchem eines oder mehrere der Wasserstoffatome durch Alkyl, Alkoxy, Halogen, Cyano, Nitro, Alkoxycarbonyl oder $C_1$–$C_8$-Alkanoyloxy ersetzt sein können, sowie Salze davon.

2. Verbindungen gemäss Anspruch 1, worin $R^2$ Diallylamino bedeutet.

3. Verbindungen gemäss Anspruch 1 oder 2, worin $R^1$ Wasserstoff und die Gruppe $-\overset{\overset{O}{\|}}{C}-R^3$ bedeutet.

4. Verbindungen gemäss Anspruch 3, worin $R^3$ Aryl, Alkoxyalkyl, die Gruppe $-C(R^7)=C(R^8)(R^9)$ oder gegebenenfalls über eine Methylengruppe gebundenes Pyridyl, Furyl oder Thienyl bedeutet.

5. Verbindungen gemäss Anspruch 4, worin $R^3$ Phenyl, Methoxymethyl, Äthoxymethyl, die Gruppe $-C(CH_3)=CH_2$ oder $-CH=C(CH_3)_2$ oder Furyl bedeutet.

6. Verbindungen gemäss Anspruch 2, worin $R^1$ Wasserstoff oder die Gruppe $-\overset{\overset{O}{\|}}{C}-R^3$ und $R^3$ Phenyl, Methoxymethyl, Äthoxymethyl, die Gruppe $-C(CH_3)=CH_2$ oder $-CH=C(CH_3)_2$ oder Furyl bedeuten.

7. N-(5-Diallylamino-2-oxo-2H-[1,2,4]oxadiazolo[2,3-a]-s-triazin-7-yl)benzamid.

8. N-(5-Diallylamino-2-oxo-2H-[1,2,4]oxadiazolo[2,3-a]-s-triazin-7-yl)-2-methoxyacetamid.

9. 2-Äthoxy-N-(5-diallylamino-2-oxo-2H-[1,2,4]-oxadiazolo[2,3-a]-s-triazin-7-yl)acetamid.

10. N-(5-Diallylamino-2-oxo-2H-[1,2,4]oxadiazolo[2,3-a]-s-triazin-7-yl)-2-methylacrylamid.

11. N-(5-Diallylamino-2-oxo-2H-[1,2,4]oxadiazolo[2,3-a]-s-triazin-7-yl)-2-furamid.

12. 7-Amino-5-diallylamino-2H-[1,2,4]oxadiazolo[2,3-a]-s-triazin-2-on.

13. N-(5-Diallylamino-2-oxo-2H-[1,2,4]oxadiazolo[2,3-a]-s-triazin-7-yl)-3-methylcrotonamid.

14. Oxadiazolotriazinderivate gemäss einem der Ansprüche 1–13 zur Verwendung als pharmazeutische Wirkstoffe.

15. Oxadiazolotriazinderivate gemäss einem der Ansprüche 1–13 zur Verwendung als Antihypertensiva und/oder Vasodilatatoren.

16. Verfahren zur Herstellung von Oxadiazolotriazinderivaten der in Anspruch 1 wiedergegebenen Formel I sowie von deren Salzen, dadurch gekennzeichnet, dass man

a) eine Verbindung der allgemeinen Formel

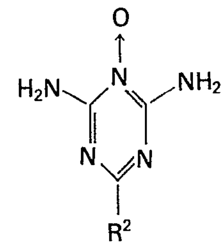

II

worin $R^2$ die in Anspruch 1 genannte Bedeutung besitzt, mit Phosgen umsetzt und gegebenenfalls eine so erhaltene Verbindung der allgemeinen Formel

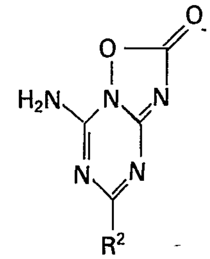

Ia

worin $R^2$ die obige Bedeutung besitzt, mit einem die Gruppe $-\overset{\overset{O}{\|}}{C}-R^3$ abgebenden Acylierungsmittel, wobei $R^3$ die in Anspruch 1 genannte Bedeutung besitzt, mit einem Chlorameisensäureester der allgemeinen Formel

$$Cl-COOR^4 \qquad III$$

worin $R^4$ die in Anspruch 1 genannte Bedeutung besitzt, oder mit einer Verbindung der allgemeinen Formel

$$XCH(R^5)COOR^6 \qquad IV$$

worin X Halogen bedeutet und $R^5$ und $R^6$ die in Anspruch 1 genannte Bedeutung besitzen, umsetzt, oder

b) zur Herstellung von Verbindungen der Formel I, worin $R^1$ die Gruppe $-COOR^4$ bedeutet und $R^2$ die obige Bedeutung besitzt, eine Verbindung der allgemeinen Formel

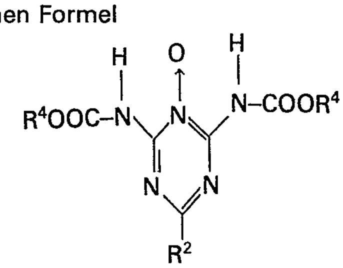

V

worin $R^2$ und $R^4$ die obige Bedeutung besitzen, cyclisiert, und

c) erwünschtenfalls eine erhaltene Verbindung der Formel I in ein Salz oder ein Salz in ein anderes Salz überführt.

17. Arzneimittel enthaltend ein Oxadiazolotriazinderivat gemäss einem der Ansprüche 1–13 oder ein pharmazeutisch verwendbares Salz

davon.

18. Antihypertensivum und/oder Vasodilatator enthaltend ein Oxadiazolotriazinderivat gemäss einem der Ansprüche 1–13 oder ein pharmazeutisch verwendbares Salz davon.

**Patentansprüche für den Vertragsstaat AT**

1. Verfahren zur Herstellung von Oxadiazolotriazinderivate der allgemeinen Formel

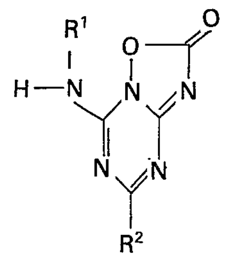

I

worin $R^1$ Wasserstoff oder eine der Gruppen

$-\overset{O}{\overset{\|}{C}}-R^3$, $-COOR^4$ und $-CH(R^5)COOR^6$ bedeutet und $R^2$ Diallylamino oder 1,2,5,6-Tetrahydropyridin-1-yl, $R^3$ Alkyl, Haloalkyl, Alkoxyalkyl, Aryloxyalkyl, Arylalkyloxyalkyl, Arylalkyl, Alkoxycarbonylalkyl, Aryl, die Gruppe $-C(R^7)=C(R^8)(R^9)$, Alkoxycarbonylalkylcarbonyl oder gegebenenfalls über eine Methylengruppe gebundenes Pyridyl, Furyl oder Thienyl, $R^4$ Alkyl, Alkoxyalkyl, Arylalkyl, Aryl oder Allyl, $R^5$ Wasserstoff oder Alkyl, $R^6$ Alkyl, $R^7$ und $R^8$ je Wasserstoff, Alkyl, Aryl, Arylalkyl, Pyridyl, Furyl oder Thienyl und $R^9$ Wasserstoff oder Methyl bedeuten, wobei die Alkyl- und Alkoxyreste 1–8 Kohlenstoffatome aufweisen und der Arylrest – allein oder in Kombination – einen ein- oder zweikernigen aromatischen Rest mit bis zu 12 Kohlenstoffatomen bedeutet, in welchem eines oder mehrere der Wasserstoffatome durch Alkyl, Alkoxy, Halogen, Cyano, Nitro, Alkoxycarbonyl oder $C_1$–$C_8$-Alkanoyloxy ersetzt sein können, sowie Salze davon, dadurch gekennzeichnet, dass man

a) eine Verbindung der allgemeinen Formel

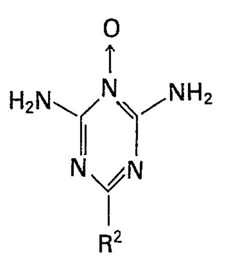

II

worin $R^2$ die in Anspruch 1 genannte Bedeutung besitzt, mit Phosgen umsetzt und gegebenenfalls eine so erhaltene Verbindung der allgemeinen Formel

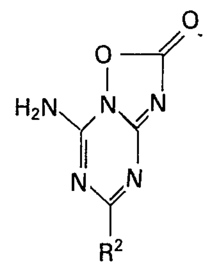

Ia

worin $R^2$ die obige Bedeutung besitzt, mit einem die Gruppe $-\overset{O}{\overset{\|}{C}}-R^3$ abgebenden Acylierungsmittel, wobei $R^3$ die in Anspruch 1 genannte Bedeutung besitzt, mit einem Chlorameisensäureester der allgemeinen Formel

$Cl-COOR^4$ III

worin $R^4$ die in Anspruch 1 genannte Bedeutung besitzt, oder mit einer Verbindung der allgemeinen Formel

$XCH(R^5)COOR^6$ IV

worin X Halogen bedeutet und $R^5$ und $R^6$ die in Anspruch 1 genannte Bedeutung besitzen, umsetzt, oder

b) zur Herstellung von Verbindungen der Formel I, worin $R^1$ die Gruppe $-COOR^4$ bedeutet und $R^2$ die obige Bedeutung besitzt, eine Verbindung der allgemeinen Formel

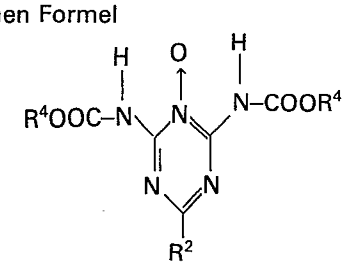

V

worin $R^2$ und $R^4$ die obige Bedeutung besitzen, cyclisiert, und

c) erwünschtenfalls eine erhaltene Verbindung der Formel I in ein Salz oder ein Salz in ein anderes Salz überführt.

2. Verfahren gemäss Anspruch 1, worin $R^2$ Diallylamino bedeutet.

3. Verfahren gemäss Anspruch 1 oder 2, worin $R^1$ Wasserstoff und die Gruppe $-\overset{O}{\overset{\|}{C}}-R^3$ bedeutet.

4. Verfahren gemäss Anspruch 3, worin $R^3$ Aryl, Alkoxyalkyl, die Gruppe $-C(R^7)=C(R^8)(R^9)$ oder gegebenenfalls über eine Methylengruppe gebundenes Pyridyl, Furyl oder Thienyl bedeutet.

5. Verfahren gemäss Anspruch 4, worin $R^3$ Phenyl, Methoxymethyl, Äthoxymethyl, die Gruppe $-C(CH_3)=CH_2$ oder $-CH=C(CH_3)_2$ oder Furyl bedeutet.

6. Verfahren gemäss Anspruch 2, worin $R^1$ Wasserstoff oder die Gruppe $-\overset{\overset{O}{\|}}{C}-R^3$ und $R^3$ Phenyl, Methoxymethyl, Äthoxymethyl, die Gruppe $-C(CH_3)=CH_2$ oder $-CH=C(CH_3)_2$ oder Furyl bedeuten.

7. Verfahren nach Anspruch 1, dadurch gekennzeichnet, dass man N-(5-Diallylamino-2-oxo-2H-[1,2,4]-oxadiazolo[2,3-a]-s-triazin-7-yl)benzamid herstellt.

8. Verfahren nach Anspruch 1, dadurch gekennzeichnet, dass man N-(5-Diallylamino-2-oxo-2H-[1,2,4]oxadiazolo[2,3-a]-s-triazin-7-yl)-2-methoxyacetamid herstellt.

9. Verfahren nach Anspruch 1, dadurch gekennzeichnet, dass man 2-Äthoxy-N-(5-diallylamino-2-oxo-2H-[1,2,4]oxadiazolo[2,3-a]-s-triazin-7-yl) acetamid herstellt.

10. Verfahren nach Anspruch 1, dadurch gekennzeichnet, dass man N-(5-Diallylamino-2-oxo-2H-[1,2,4]oxadiazolo[2,3-a]-s-triazin-7-yl)-2-methylacrylamid herstellt.

11. Verfahren nach Anspruch 1, dadurch gekennzeichnet, dass man N-(5-Diallylamino-2-oxo-2H-[1,2,4]oxadiazolo[2,3-a]-s-triazin-7-yl)-2-furamid herstellt.

12. Verfahren nach Anspruch 1, dadurch gekennzeichnet, dass man 7-Amino-5-diallyl-amino-2H-[1,2,4]oxadiazolo[2,3-a]-s-triazin-2-on herstellt.

13. Verfahren nach Anspruch 1, dadurch gekennzeichnet, dass man N-(5-Diallylamino-2-oxo-2H-[1,2,4]oxadiazolo[2,3-a]-s-triazin-7-yl)-3-methylcrotonamid herstellt.

**Claims for the contracting states BE, CH, DE, FR, GB, IT, LU, NL, SE**

1. Oxadiazolotriazine derivatives of the general formula

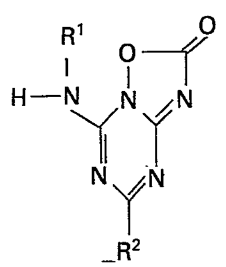

I

wherein $R^1$ signifies hydrogen or one of the groups $-\overset{\overset{O}{\|}}{C}-R^3$, $-COOR^4$ and $-CH(R^5)COOR^6$ and $R^2$ signifies diallylamino or 1,2,5,6-tetrahydropyridin-1-yl, $R^3$ signifies alkyl, haloalkyl, alkoxyalkyl, aryloxyalkyl, arylalkyloxyalkyl, arylalkyl, alkoxycarbonylalkyl, aryl, the group $-C(R^7)=C(R^8)(R^9)$, alkoxycarbonylalkylcarbonyl or pyridyl, furyl or thienyl optionally bound via a methylene group, $R^4$ signifies alkyl, alkoxyalkyl, arylalkyl, aryl or allyl, $R^5$ signifies hydrogen or alkyl, $R^6$ signifies alkyl, $R^7$ and $R^8$ each signify hydrogen, alkyl, aryl, arylalkyl, pyridyl, furyl or thienyl and $R^9$ signifies hydrogen or methyl, the alkyl and alkoxy residues having 1–8 carbon atoms and the aryl residue – alone or in combination – signifying a mono- or dinuclear aromatic residue with up to 12 carbon atoms in which one or more of the hydrogen atoms can be replaced by alkyl, alkoxy, halogen, cyano, nitro, alkoxycarbonyl or $C_1$–$C_8$-alkanoyloxy, as well as salts thereof.

2. Compounds according to claim 1, wherein $R^2$ signifies diallylamino.

3. Compounds according to claim 1 or 2, wherein $R^1$ signifies hydrogen and the group $-\overset{\overset{O}{\|}}{C}-R^3$.

4. Compounds according to claim 3, wherein $R^3$ signifies aryl, alkoxyalkyl, the group $-C(R^7)=C(R^8)(R^9)$ or pyridyl, furyl or thienyl optionally bound via a methylene group.

5. Compounds according to claim 4, wherein $R^3$ signifies phenyl, methoxymethyl, ethoxymethyl, the group $-C(CH_3)=CH_2$ or $-CH=C(CH_3)_2$ or furyl.

6. Compounds according to claim 2, wherein $R^1$ signifies hydrogen or the group $-\overset{\overset{O}{\|}}{C}-R^3$ and $R^3$ signifies phenyl, methoxymethyl, ethoxymethyl, the group $-C(CH_3)=CH_2$ or $-CH=C(CH_3)_2$ or furyl.

7. N-(5-Diallylamino-2-oxo-2H-[1,2,4]oxadiazolo[2,3-a]-s-triazin-7-yl)benzamide.

8. N-(5-Diallylamino-2-oxo-2H-[1,2,4]oxadiazolo[2,3-a]-s-triazin-7-yl)-2-methoxyacetamide.

9. 2-Ethoxy-N-(5-diallylamino-2-oxo-2H-[1,2,4]-oxadiazolo[2,3-a]-s-triazin-7-yl)acetamide.

10. N-(5-Diallylamino-2-oxo-2H-[1,2,4]oxadiazolo[2,3-a]-s-triazin-7-yl)-2-methylacrylamide.

11. N-(5-Diallylamino-2-oxo-2H-[1,2,4]oxadiazolo[2,3-a]-s-triazin-7-yl)-2-furamide.

12. 7-Amino-5-diallylamino-2H-[1,2,4]oxadiazolo[2,3-a]-s-triazin-2-one.

13. N-(5-Diallylamino-2-oxo-2H-[1,2,4]oxadiazolo[2,3-a]-s-triazin-7-yl)-3-methylcrotonamide.

14. Oxadiazolotriazine derivatives according to any one of claims 1–13 for use as pharmaceutically active substances.

15. Oxadiazolotriazine derivatives according to any one of claims 1–13 for use as antihypertensives and/or vasodilators.

16. Process for the manufacture of oxadiazolotriazine derivatives of formula I inidcated in claim 1 as well as of their salts, characterized by

a) reacting a compound of the general formula

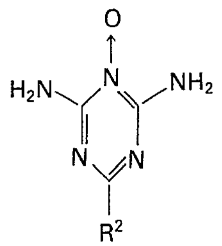

II

wherein $R^2$ has the significance given in claim 1, with phosgene and optionally reacting a thus-obtained compound of the general formula

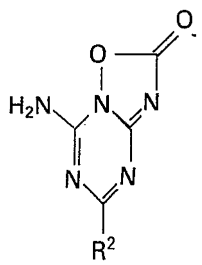

Ia

wherein $R^2$ has the above significance, with an acylating agent providing the group 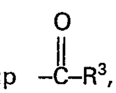, wherein $R^3$ has the significance given in claim 1, with a chloroformic acid ester of the general formula

$Cl{-}COOR^4$ III

wherein $R^4$ has the significance given in claim 1, or with a compound of the general formula

$XCH(R^5)COOR^6$ IV

wherein X signifies halogen and $R^5$ and $R^6$ have the significance given in claim 1, or

b) for the manufacture of compounds of formula I wherein $R^1$ signifies the group $-COOR^4$ and $R^2$ has the above significance, cyclizing a compound of the general formula

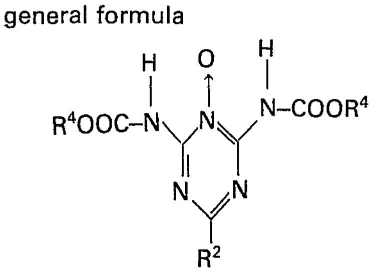

V

wherein $R^2$ und $R^4$ have the above significance, and

c) if desired, converting a compound of formula I obtained into a salt or converting a salt into a different salt.

17. Medicament containing an oxadiazolotriazine derivative according to any one of claims 1–13 or a pharmaceutically usable salt thereof.

18. Antihypertensive and/or vasodilator containing an oxadiazolotriazine derivative according to any one of claims 1–13 or a pharmaceutically usable salt thereof.

**Claims for the contracting state AT**

1. Process for the manufacture of oxadiazolotriazin derivatives of the general formula

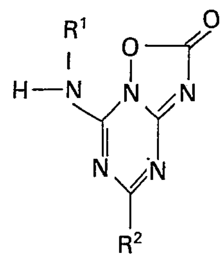

I

wherein $R^1$ signifies hydrogen or one of the groups 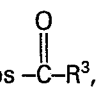, $-COOR^4$ and $-CH(R^5)COOR^6$ and $R^2$ signifies diallylamino or 1,2,5,6-tetrahydropyridin-1-yl, $R^3$ signifies alkyl, haloalkyl, alkoxyalkyl, aryloxyalkyl, arylalkyloxyalkyl, arylalkyl, alkoxycarbonylalkyl, aryl, the group $-C(R^7)=C(R^8)(R^9)$, alkoxycarbonylalkylcarbonyl or pyridyl, furyl or thienyl optionally bound via a methylene group, $R^4$ signifies alkyl, alkoxyalkyl, arylalkyl, aryl or allyl, $R^5$ signifies hydrogen or alkyl, $R^6$ signifies alkyl, $R^7$ and $R^8$ each signify hydrogen, alkyl, aryl, arylalkyl, pyridyl, furyl or thienyl and $R^9$ signifies hydrogen or methyl, the alkyl and alkoxy residues having 1–8 carbon atoms and the aryl residue – alone or in combination – signifying a mono- or dinuclear aromatic residue with up to 12 carbon atoms in which one or more of the hydrogen atoms can be replaced by alkyl, alkoxy, halogen, cyano, nitro, alkoxycarbonyl or $C_1$–$C_8$-alkanoyloxy, as well as salts thereof, characterized by

a) reacting a compound of the general formula

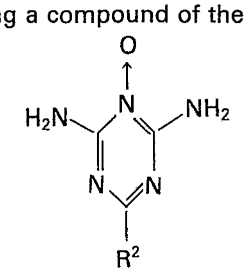

II

wherein $R^2$ has the significance given in claim 1, with phosgene and optionally reacting a thus-obtained compound of the general formula

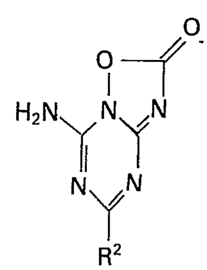

Ia

wherein $R^2$ has the above significance, with an

acylating agent providing the group $-\overset{O}{\overset{\|}{C}}-R^3$, wherein $R^3$ has the significance given in claim 1, with a chloroformic acid ester of the general formula

$Cl-COOR^4$ III

wherein $R^4$ has the significance given in claim 1, or with a compound of the general formula

$XCH(R^5)COOR^6$ IV

wherein X signifies halogen and $R^5$ and $R^6$ have the significance given in claim 1, or

b) for the manufacture of compounds of formula I wherein $R^1$ signifies the group $-COOR^4$ and $R^2$ has the above significance, cyclizing a compound of the general formula

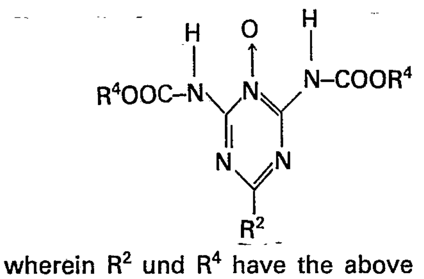

V

wherein $R^2$ und $R^4$ have the above significance, and

c) if desired, converting a compound of formula I obtained into a salt or converting a salt into a different salt.

2. Process according to claim 1, wherein $R^2$ signifies diallylamino.

3. Process according to claim 1 or 2, wherein $R^1$ signifies hydrogen and the group $-\overset{O}{\overset{\|}{C}}-R^3$.

4. Process according to claim 3, wherein $R^3$ signifies aryl, alkoxyalkyl, the group $-C(R^7)=C(R^8)(R^9)$ or pyridyl, furyl or thienyl optionally bound via a methylene group.

5. Process according to claim 4, wherein $R^3$ signifies phenyl, methoxymethyl, ethoxymethyl, the group $-C(CH_3)=CH_2$ or $-CH=C(CH_3)_2$ or furyl.

6. Process according to claim 2, wherein $R^1$ signifies hydrogen or the group $-\overset{O}{\overset{\|}{C}}-R^3$ and $R^3$ signifies phenyl, methoxymethyl, ethoxymethyl, the group $-C(CH_3)=CH_2$ or $-CH=C(CH_3)_2$ or furyl.

7. Process according to claim 1, characterized in that N-(5-diallylamino-2-oxo-2H-[1,2,4]oxadiazolo[2,3-a]-s-triazin-7-yl)benzamide is manufactured.

8. Process according to claim 1, characterized in that N-(5-diallylamino-2-oxo-2H-[1,2,4]oxadiazolo[2,3-a]-s-triazin-7-yl)-2-methoxyacetamide is manufactured.

9. Process according to claim 1, characterized in that 2-ethoxy-N-(5-diallylamino-2-oxo-2H-[1,2,4]-oxadiazolo[2,3-a]-s-triazin-7-yl)acetamide is manufactured.

10. Process according to claim 1, characterized in that N-(5-diallylamino-2-oxo-2H-[1,2,4]oxadiazolo[2,3-a]-s-triazin-7-yl)-2-methylacrylamide is manufactured.

11. Process according to claim 1, characterized in that N-(5-Diallylamino-2-oxo-2H-[1,2,4]oxadiazolo[2,3-a]-s-triazin-7-yl)-2-furamide is manufactured.

12. Process according to claim 1, characterized in that 7-Amino-5-diallylamino-2H-[1,2,4]oxadiazolo[2,3-a]-s-triazin-2-one is manufactured.

13. Process according to claim 1, characterized in that N-(5-Diallylamino-2-oxo-2H-[1,2,4]oxadiazolo[2,3-a]-s-triazin-7-yl)-3-methylcrotonamide is manufactured.

**Revendications pour les états contractants BE, CH, DE, FR, GB, IT, LU, NL, SE**

1. Dévrivés de l'oxadiazolotriazine de formule générale:

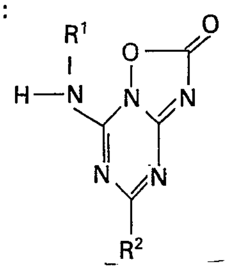

I

dans laquelle $R^1$ représente l'hydrogène ou l'un des groupes $-\overset{O}{\overset{\|}{C}}-R^3$, $-COOR^4$ et $-CH(R^5)COOR^6$ et $R^2$ représente un groupe diallylamino ou 1,2,5,6-tétrahydropyridine-1-yle, $R^3$ représente un groupe alkyle, halogénoalkyle, alcoxyalkyle, aryloxyalkyle, arylalkyloxyalkyle, arylalkyle, alkoxycarbonylealkyle, aryle, le groupe $-C(R^7)=C(R^8)(R^9)$, un groupe alcoxycarbonylalkylcarbonyle ou un groupe pyridyle, furyle ou thiényle éventuellement relié par l'intermédiaire d'un groupe méthylene, $R^4$ représente un groupe alkyle, alcoxyalkyle, arylalkyle, aryle ou allyle, $R^5$ représente l'hydrogène ou un groupe alkyle, $R^6$ représente un groupe alkyle, $R^7$ et $R^8$ représentent chacun l'hydrogène, un groupe alkyle, aryle, arylalkyle, pyridyle, furyle ou thiényle, et $R^9$ représente l'hydrogène ou un groupe méthyle, les groupes alkyle et alcoxy contenant 1 à 8 atomes de carbone et le reste aryle – seul ou en combination – étant un reste aromatique mono- ou dicyclique contenant jusqu'à 12 atomes de carbone et dans lequel un ou plusieurs des atomes d'hydrogène peuvent être remplacés par des groupes alkyle, alcoxy, des atomes d'halogène, des groupes cyano, nitro, alcoxycarbonyle ou alcanoyloxy en $C_1$–$C_8$, et leurs sels.

2. Composés selon la revendication 1, dans les-

quels $R^2$ représente un groupe diallylamino.

3. Composés selon la revendication 1 ou 2, dans lesquels $R^1$ représente l'hydrogène et le groupe

O
||
$-C-R^3$.

4. Composés selon la revendication 3, dans lesquels $R^3$ représente un groupe aryle, alcoxyalkyle, le groupe $-C(R^7)=C(R^8)(R^9)$ ou un groupe pyridyle, furyle ou thiényle éventuellement relié par l'intermédiaire d'un groupe méthylène.

5. Composés selon la revendication 4, dans lesquels $R^3$ représente un groupe phényle, méthoxyméthyle, éthoxyméthyle, le groupe $-C(CH_3)=CH_2$ ou $-CH=C(CH_3)_2$ ou furyle.

6. Composés selon la revendication 2, dans lesquels $R^1$ représente l'hydrogène ou le groupe

O
||
$-C-R^3$, et $R^3$ représente un groupe phényle, méthoxyméthyle, éthoxyméthyle, le groupe $-C(CH_3)=CH_2$ ou $-CH=C(CH_3)_2$ ou un groupe furyle.

7. Le N-(5-diallylamino-2-oxo-2H-[1,2,4]oxadiazolo[2,3-a]-s-triazine-7-yl)benzamide.

8. Le N-(5-diallylamino-2-oxo-2H-[1,2,4]oxadiazolo[2,3-a]-s-triazine-7-yl)-2-méthoxyacétamide.

9. Le 2-éthoxy-N-(5-diallylamino-2-oxo-2H-[1,2,4]oxadiazolo[2,3-a]-s-triazine-7-yl)acétamide.

10. Le N-(5-diallylamino-2-oxo-2H-[1,2,4]oxadiazolo[2,3-a]-s-triazine-7-yl)-2-méthylacrylamide.

11. Le N-(5-diallylamino-2-oxo-2H-[1,2,4]oxadiazolo[2,3-a]-s-triazine-7-yl)-2-furamide.

12. La 7-amino-5-diallylamino-2H-[1,2,4]oxadiazolo[2,3-a]-s-triazine-2-one.

13. Le N-(5-diallylamino-2-oxo-2H-[1,2,4]oxadiazolo[2,3-a]-s-triazine-7-yl)-3-méthylcrotonamide.

14. Dérivés de l'oxadiazolotriazine selon l'une quelconque des revendications 1 à 13, pour l'utilisation en tant que substances actives pharmaceutiques.

15. Dérivés de l'oxadiazolotriazine selon l'une quelconque des revendications 1 à 13 pour l'utilisation en tant qu'anti-hypertensifs ou vasodilatateurs.

16. Procédé de préparation des dérivés de l'oxadiazolotriazine de formule I indiqué dans la revendication 1 et de leurs sels, caractérisé en ce que

a) on fait réagir un composé de formule générale

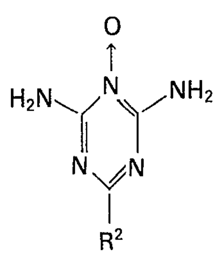

II

dans laquelle $R^2$ a la signification indiquée dans la revendication 1, avec le phosgène, et le cas échéant, on fait réagir un composé ainsi obtenu, de formule générale

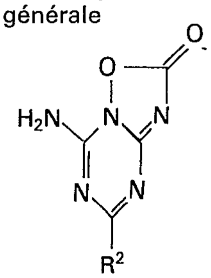

Ia

dans laquelle $R^2$ a la signification indiquée ci-dessus, avec un agent acylant fournissant le groupe

O
||
$-C-R^3$, $R^3$ ayant la signification indiquée dans la revendication 1, avec un ester chloroformique de formule générale

$Cl-COOR^4$ III

dans laquelle $R^4$ a la signification indiquée dans la revendication 1, ou avec un composé de formule générale

$XCH(R^5)COOR^6$ IV

dans laquelle X représente un halogène, et $R^5$ et $R^6$ ont les significations indiquées dans la revendication 1, ou bien

b) pour préparer les composés de formule I dans laquelle $R^1$ représente le groupe $-COOR^4$ et $R^2$ a la signification indiquée ci-dessus, on cyclise un composé de formule générale:

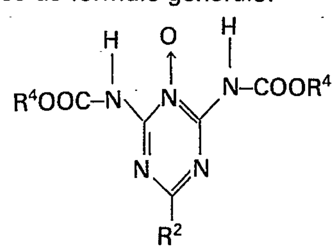

V

dans laquelle $R^2$ et $R^4$ ont les significations indiquées ci-dessus, et

c) si on le désire, on convertit un composé obtenu, répondant à la formule I, en un sel, ou un sel en un autre sel.

17. Médicament contenant un dérivé de l'oxadiazolotriazine selon l'une quelconque des revendications 1 à 13 ou un sel d'un tel dérivé acceptable pour l'usage pharmaceutique.

18. Anti-hypertensifs et/ou vaso-dilatateurs contenant un dérivé de l'oxadiazolotriazine selon l'une quelconque des revendications 1 à 13, ou un sel d'un tel dérivé acceptable pour l'usage pharmaceutique.

**Revendications pour l'état contractant AT**

1. Procédé de préparation dévrivés de l'oxadiazolotriazine de formule générale:

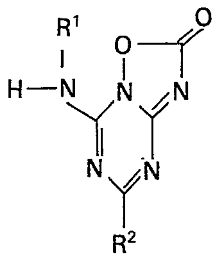

I

dans laquelle $R^1$ représente l'hydrogène ou l'un des groupes $-\overset{O}{\overset{\|}{C}}-R^3$, $-COOR^4$ et $-CH(R^5)COOR^6$ et $R^2$ représente un groupe diallylamino ou 1,2,5,6-tétrahydropyridine-1-yle, $R^3$ représente un groupe alkyle, halogénoalkyle, alcoxyalkyle, aryloxyalkyle, arylalkyloxyalkyle, arylalkyle, alkoxycarbonyl-ealkyle, aryle, le groupe $-C(R^7)=C(R^8)(R^9)$, un groupe alcoxycarbonylalkylcarbonyle ou un groupe pyridyle, furyle ou thiényle éventuellement relié par l'intermédiaire d'un groupe méthylene, $R^4$ représente un groupe alkyle, alcoxyalkyle, arylalkyle, aryle ou allyle, $R^5$ représente l'hydrogène ou un groupe alkyle, $R^6$ un groupe alkyle, $R^7$ et $R^8$ représentent chacun l'hydrogène, un groupe alkyle, aryle, arylalkyle, pyridyle, furyle ou thiényle, et $R^9$ représente l'hydrogène ou un groupe méthyle, les groupes alkyle et alcoxy contenant 1 à 8 atomes de carbone et le reste aryle – seul ou en combination – étant un reste aromatique mono- ou di-cyclique contenant jusqu'à 12 atomes de carbone et dans lequel un ou plusieurs des atomes d'hydrogène peuvent être remplacés par des groupes alkyle, alcoxy, des atomes d'halogène, des groupes cyano, nitro, alcoxycarbonyle ou alcanoyloxy en $C_1$–$C_8$, et leurs sels, caractérisé en ce que

a) on fait réagir un composé de formule générale

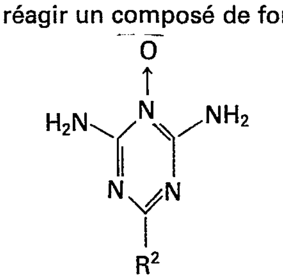

II

dans laquelle $R^2$ a la signification indiquée dans la revendication 1, avec le phosgène, et le cas échéant, on fait réagir un composé ainsi obtenu, de formule générale

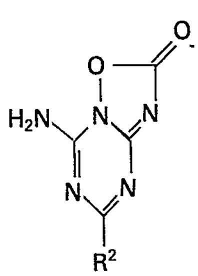

Ia

dans laquelle $R^2$ a la signification indiqué ci-dessus, avec un agent acylant fournissant le groupe

$-\overset{O}{\overset{\|}{C}}-R^3$, $R^3$ ayant la signification indiquée dans la revendication 1, avec un ester chloroformique de formule générale

$Cl-COOR^4$ III

dans laquelle $R^4$ a la signification indiquée dans la revendication 1, ou avec un composé de formule générale

$XCH(R^5)COOR^6$ IV

dans laquelle X représente un halogène, et $R^5$ et $R^6$ ont les significations indiquées dans la revendication 1, ou bien

b) pour préparer les composés de formule I dans laquelle $R^1$ représente le groupe $-COOR^4$ et $R^2$ a la signification indiquée ci-dessus, on cyclise un composé de formule générale:

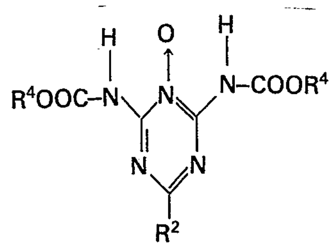

V

dans laquelle $R^2$ et $R^4$ ont les significations indiquées ci-dessus, et

c) si on le désire, on convertit un composé obtenu, répondant à la formule I, en un sel, ou un sel en un autre sel.

2. Procédé selon la revendication 1, dans lequel $R^1$ représente le groupe diallylamino.

3. Procédé selon la revendication 1 ou 2, dans lequel $R^1$ représente l'hydrogène et le groupe

$-\overset{O}{\overset{\|}{C}}-R^3$.

4. Procédé selon la revendication 3, dans lequel $R^3$ représente un groupe aryle, alcoxyalkyle, le groupe $-C(R^7)=C(R^8)(R^9)$ ou en groupe pyridyle, furyle ou thiényle éventuellement relié par l'intermédiaire d'un groupe méthylène.

5. Procédé selon la revendication 4, dans lequel $R^3$ représente un groupe phényle, méthoxyméthyle, éthoxyméthyle, le groupe $-C(CH_3)=CH_2$ ou $-CH=C(CH_3)_2$ ou un groupe furyle.

6. Procédé selon la revendication 2, dans lequel $R^1$ représente l'hydrogène ou le groupe

$-\overset{O}{\overset{\|}{C}}-R^3$, et $R^3$ un groupe phényle, méthoxyméthyle,

éthoxyméthyle, le groupe $-C(CH_3)=CH_2$ ou $-CH=C(CH_3)_2$ ou un groupe furyle.

7. Procédé selon la revendication 1, caractérisé en ce que l'on prépare le N-(5-Diallylamino-2-oxo-2H-[1,2,4]oxadiazolo[2,3-a]-s-triazine-7-yl)benzamide.

8. Procédé selon la revendication 1, caractérisé en ce que l'on prépare le N-(5-diallylamino-2-oxo-2H-[1,2,4]oxadiazolo[2,3-a]-s-triazine-7-yl)-2-méthoxyacétamide.

9. Procédé selon la revendication 1, caractérisé en ce que l'on prépare le 2-éthoxy-N-(5-diallylamino-2-oxo-2H-[1,2,4]oxadiazolo[2,3-a]-s-triazine-7-yl)acétamide.

10. Procédé selon la revendication 1, caractérisé en ce que l'on prépare le N-(5-diallylamino-2-oxo-2H-[1,2,4]oxadiazolo[2,3-a]-s-triazine-7-yl)-2-méthylacrylamide.

11. Procédé selon la revendication 1, caractérisé en ce que l'on prépare le N-(5-diallylamino-2-oxo-2H-[1,2,4]oxadiazolo[2,3-a]-s-triazine-7-yl)-2-furamide.

12. Procédé selon la revendication 1, caractérisé en ce que l'on prépare la 7-amino-5-diallylamino-2H-[1,2,4]oxadiazolo[2,3-a]-s-triazine-2-one.

13. Procédé selon la revendication 1, caractérisé en ce que l'on prépare le N-(5-diallylamino-2-oxo-2H-[1,2,4]oxadiazolo[2,3-a]-s-triazine-7-yl)-3-méthylcrotonamide.